# EUROPEAN PATENT APPLICATION

(11) **EP 4 678 010 A1**
(43) Date of publication of application: **14.01.2026**
(21) Application number: 24187233.2
(22) Date of filing: 08.07.2024
(51) Int. Cl.: A23J 3/22, A23L 13/40, A23L 27/26, A23L 31/15, C07K 14/39, C07K 14/795, C07K 14/805, C12N 15/63, C12N 15/81, C12R 1/84

(54) **FLAVOUR COMPOSITION COMPRISING A HEME PROTEIN, PREFERABLY MYOGLOBIN EXPRESSED IN YEAST**

(71) Applicant: Paleo B.V., 3290 Diest (BE)
(72) Inventor: Sanctorum, Hermes, 3290 Diest (BE); de Jong, Andy, 3290 Diest (BE); Lauwers, Elsa, 3290 Diest (BE)
(74) Representative: Nederlandsch Octrooibureau

(57) **Abstract**

This invention relates to a flavour composition comprising a hemeprotein for modifying the flavour of a foodstuff or a beverage, use of said flavour composition, a method for improving the flavour of a foodstuff or a beverage, and a method for preparing the flavour composition.

## Description

### Field

This invention relates to a flavour composition comprising a hemeprotein for modifying the flavour of a foodstuff or a beverage, use of said flavour composition, a method for improving the flavour of a foodstuff or a beverage, and a method for preparing the flavour composition.

### Background

Food flavourings are products added to food to give them a different or stronger taste and/or smell. They can be produced in different ways such as by extraction from plants or from other material of vegetable, animal or microbiological origin. Flavourings can also be synthesized or obtained through a variety of different processes.

Consumers have individual preferences for flavors and perceived experience. Some consumers seek out flavor ingredients that can provide meat-like taste and flavour and healthier substance at the same time.

Hence, there is a need in the art for optimizing the quality and source of flavour composition.

### Description

A flavoring is a volatile additive that improves the taste or smell of food. They work primarily via the sense of smell. In legislation, substances that exclusively have a sweet, sour or salty taste are not considered flavorings. These usually include flavor enhancers, sweeteners, acidulants and salt substitutes.

In a first aspect, the present invention provides a flavour composition comprising a hemeprotein for modifying the flavour of a foodstuff or a beverage.

### 1. Hemeproteins

In an embodiment, the flavour composition of the present invention comprises a hemeprotein. Hemeproteins are a large class of metalloproteins containing a heme prosthetic group. The heme group confers functionality, which can include oxygen carrying, oxygen reduction, electron transfer, and other processes. Heme is bound to the protein either covalently or noncovalently or both. Notable hemeproteins include hemoglobin, myoglobin, cytochrome P450 enzyme, catalase, and peroxidase, which play vital roles in diverse cellular functions. Currently, these hemoproteins have been extensively applied in the fields of food, medicine, high-cell-density fermentation, and biocatalysts.

As used herein, a hemeprotein can refer to any protein or protein subunit that is capable of covalently or noncovalently binding a heme moiety. A hemeprotein as used herein is preferably found to elicit oxygen binding activity or to carry an atom of iron bound to a heme. For example, the oxygen binding activity may be assessed by determining the ratio of oxygen-bound versus non-oxygen-bound myoglobin using spectrophotometry, such as the measurement and calculation method according to Tang et al. (Krzywicki revisited: equations for spectrophotometric determination of myoglobin redox forms in aqueous meat extract. J Food Sci 2004;69(9):C717-C720). For example, the activity of carrying an atom of iron bound to a heme may be assessed by detecting the absorbance peak near 400 nm (the so called Soret band) caused by the presence of heme iron using spectrophotometry, or by using mass spectrometry.

In some embodiments, the hemeprotein comprises a heme group. The heme group consists of an organic component protoporphyrin as well as an inorganic component that consists of an iron atom. There is not a single ubiquitous regulatory mechanism for heme biosynthesis. In mammalian cells, heme is predominantly synthesized in bone marrow (i.e., in erythroblasts and reticulocytes that still contain mitochondria) and liver cells. In Pichia pastoris, heme biosynthesis begins with L-glutamate and proceeds through 5-amino-levulinate, a universal tetrapyrrole precursor, to uroporphyrinogen III. Uroporphyrinogen III is converted to protoheme IX which further produce heme, by incorporating iron, in the subsequent reactions. Heme-containing polypeptides may transport or store oxygen. Some examples of known heme-containing protein or polypeptide include hemoglobin, myoglobin, neuroglobin, cytoglobin and leghemoglobin, androglobin, a globin E, a globin X, a globin Y, a flavohemoglobin, Hell's gate globin I, an erythrocruorin, a bèta hemoglobin, an alpha hemoglobin, a protoglobin, a cyanoglobin, a cytoglobin, a histoglobin, a chlorocruorin, a truncated hemoglobin (e.g., HbN or HbO), a truncated 2/2 globin, a hemoglobin 3 (e.g., Glb3), a cytochrome, or a peroxidase.

In some embodiments, the hemeprotein is an animal hemeprotein. In some embodiments, the hemeprotein is from a mammoth, preferably from a woolly mammoth or a steppe mammoth. In some embodiments, the hemeprotein is from pig. In some embodiments, the hemeprotein is from sheep. In some embodiments, the hemeprotein is from cow. In some embodiments, the hemeprotein is from chicken. In some embodiments, the hemeprotein is from rabbit. In some embodiment, the hemeprotein is from bovine. In some embodiments, the hemeprotein is from a mouse. In some embodiments, the hemeprotein is from a rat. In some embodiments, the hemeprotein is from tuna. In some embodiments, the hemeprotein is from salmon. In some embodiments, the hemeprotein is from a blue bonito. In some embodiments, the hemeprotein is from a sword fish.

In some embodiments, the hemeprotein is a hemeprotein obtained from microbial fermentation. In an embodiment, the hemeprotein is derived from an animal. In preferred embodiments, the hemeprotein is derived from an animal, preferably from steppe mammoth, woolly mammoth, pig, sheep, cow, chicken, rabbit, bovine, mouse, rat, tuna, salmon, blue bonito, or sword fish.

In an embodiment, the hemeprotein is a metalloprotein. In an embodiment, the hemeprotein is a globin, preferably hemoglobin, myoglobin, neuroglobin, cytoglobin or leghemoglobin, more preferably myoglobin or hemoglobin, most preferably myoglobin. Preferred hemeproteins are also discussed in WO 2022/144434, incorporated herein by reference.

### 2. Myoglobin

In an embodiment, there is provided a flavour composition for modifying the flavour of a foodstuff or a beverage comprising a hemeprotein according to the present invention, wherein the hemeprotein is a myoglobin.

Myoglobin is a relatively small globular protein of about 17 kDa, found in heart and skeletal muscles. It carries a single heme group with an atom of iron capable of reversible oxygen binding, allowing myoglobin to transport oxygen from the cell surface to mitochondria. Myoglobin contains 153 amino acids and as with other globins, consists of eight alpha helices connected by loops. Myoglobin contains a porphyrin ring with an iron at its center. A proximal histidine group (His-93) is attached directly to iron, and a distal histidine group (His-64) hovers near the opposite face. Myoglobin is an iron- and oxygen-binding protein found in the cardiac and skeletal muscle tissue of vertebrates in general and in almost all mammals. Muscle cells use myoglobin to accelerate oxygen diffusion and act as localized oxygen reserves for times of intense respiration.

In an embodiment, the myoglobin may be derived from steppe mammoth (Mammuthus trogontherii), woolly mammoth (Mammuthus primigenius), pig, sheep, cow, chicken, rabbit, bovine, mouse, rat, tuna, salmon, blue bonito, or sword fish. Myoglobin amino acid sequences from steppe mammoth, woolly mammoth, pig, sheep, cow, chicken, rabbit, bovine, mouse, rat, tuna, salmon, blue bonito, or sword fish have been disclosed later on by a given SEQ ID NO:1-14. In a preferred embodiment, the myoglobin may be derived from steppe mammoth (Mammuthus trogontherii) or woolly mammoth (Mammuthus primigenius).

In an embodiment, the myoglobin is derived from steppe mammoth, woolly mammoth, pig, sheep, cow, chicken, rabbit, bovine, mouse, rat, tuna, salmon, blue bonito, or sword fish by addition, insertion, deletion and/or substitution of at least one amino acid. Addition, insertion, deletion and/or substitutions of two, three, four, five, six, seven, eight, nine or ten amino acids is also contemplated by the invention. Such myoglobin derived from steppe mammoth, woolly mammoth, pig, sheep, cow, chicken, rabbit, bovine, mouse, rat, tuna, salmon, blue bonito, or sword fish may also exert at least a detectable level of an activity of a myoglobin as explained later herein.

In an embodiment, the myoglobin is a steppe mammoth myoglobin, woolly mammoth myoglobin, pig myoglobin, sheep myoglobin, cow myoglobin, chicken myoglobin, rabbit myoglobin, bovine myoglobin, mouse myoglobin, rat myoglobin, tuna myoglobin, salmon myoglobin, blue bonito myoglobin, or sword fish myoglobin or a myoglobin derived from any of these myoglobins.

In an embodiment, the myoglobin is steppe mammoth myoglobin (such as SEQ ID NO: 1) or derived therefrom. In an embodiment, the myoglobin is woolly mammoth myoglobin (such as SEQ ID NO: 2) or derived therefrom. In an embodiment, the myoglobin is pig myoglobin (such as SEQ ID NO: 3) or derived therefrom. In an embodiment, the myoglobin is sheep myoglobin (such as SEQ ID NO: 4) or derived therefrom. In an embodiment, the myoglobin is cow myoglobin (such as SEQ ID NO: 5) or derived therefrom. In an embodiment, the myoglobin is chicken myoglobin (such as SEQ ID NO: 6) or derived therefrom. In an embodiment, the myoglobin is rabbit myoglobin (such as SEQ ID NO: 7) or derived therefrom. In an embodiment, the myoglobin is bovine myoglobin (such as SEQ ID NO: 8) or derived therefrom. In an embodiment, the myoglobin is mouse myoglobin (such as SEQ ID NO: 9) or derived therefrom. In an embodiment, the myoglobin is rat myoglobin (such as SEQ ID NO: 10) or derived therefrom. In an embodiment, the myoglobin is tuna myoglobin (such as SEQ ID NO: 11) or derived therefrom. In an embodiment, the myoglobin is salmon myoglobin (such as SEQ ID NO: 12) or derived therefrom. In an embodiment, the myoglobin is blue bonito myoglobin (such as SEQ ID NO: 13) or derived therefrom. In an embodiment, the myoglobin is sword fish myoglobin (such as SEQ ID NO: 14) or derived therefrom.

In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with any one of the SEQ ID NOs: 1-14. In an embodiment, the myoglobin is represented by a sequence having at least 95% identity with any one of SEQ ID NO: 1 to 14.

In an embodiment, the myoglobin is steppe mammoth myoglobin (SEQ ID NO: 1). In an embodiment, the myoglobin is woolly mammoth myoglobin (SEQ ID NO: 2). In an embodiment, the myoglobin is pig myoglobin (SEQ ID NO: 3). In an embodiment, the myoglobin is sheep myoglobin (SEQ ID NO: 4). In an embodiment, the myoglobin is cow myoglobin (SEQ ID NO: 5). In an embodiment, the myoglobin is chicken myoglobin (SEQ ID NO: 6). In an embodiment, the myoglobin is rabbit myoglobin (SEQ ID NO: 7). In an embodiment, the myoglobin is bovine myoglobin (SEQ ID NO: 8). In an embodiment, the myoglobin is mouse myoglobin (SEQ ID NO: 9). In an embodiment, the myoglobin is rat myoglobin (SEQ ID NO: 10). In an embodiment, the myoglobin is tuna myoglobin (SEQ ID NO: 11). In an embodiment, the myoglobin is salmon myoglobin (SEQ ID NO: 12). In an embodiment, the myoglobin is blue bonito myoglobin (SEQ ID NO: 13). In an embodiment, the myoglobin is sword fish myoglobin (SEQ ID NO: 14).

In an embodiment, the myoglobin is a myoglobin obtained from microbial fermentation, preferably wherein the microbial fermentation comprises the extracellular secretion of the myoglobin.

In an embodiment, the myoglobin is derived from steppe mammoth myoglobin (SEQ ID NO: 1). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 1. A nucleic acid encoding said myoglobin which is derived from steppe mammoth myoglobin (SEQ ID NO:1) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:15.

In an embodiment, the myoglobin is derived from woolly mammoth myoglobin (SEQ ID NO: 2). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 2. A nucleic acid encoding said myoglobin which is derived from woolly mammoth myoglobin (SEQ ID NO: 2) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:16.

In an embodiment, the myoglobin is derived from pig myoglobin (SEQ ID NO: 3). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 3. A nucleic acid encoding said myoglobin which is derived from pig myoglobin (SEQ ID NO: 3) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:17.

In an embodiment, the myoglobin is derived from Sheep myoglobin (SEQ ID NO: 4). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 4. A nucleic acid encoding said myoglobin which is derived from Sheep myoglobin (SEQ ID NO: 4) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:18.

In an embodiment, the myoglobin is derived from cow myoglobin (SEQ ID NO: 5). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 5. A nucleic acid encoding said myoglobin which is derived from cow myoglobin (SEQ ID NO: 5) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:19.

In an embodiment, the myoglobin is derived from chicken myoglobin (SEQ ID NO: 6). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 6. A nucleic acid encoding said myoglobin which is derived from chicken myoglobin (SEQ ID NO: 6) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:20.

In an embodiment, the myoglobin is derived from rabbit myoglobin (SEQ ID NO: 7). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 7. A nucleic acid encoding said myoglobin which is derived from rabbit myoglobin (SEQ ID NO: 7) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:21.

In an embodiment, the myoglobin is derived from bovine myoglobin (SEQ ID NO: 8). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 8. A nucleic acid encoding said myoglobin which is derived from bovine myoglobin (SEQ ID NO: 8) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:22.

In an embodiment, the myoglobin is derived from mouse myoglobin (SEQ ID NO: 9). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 9. A nucleic acid encoding said myoglobin which is derived from mouse myoglobin (SEQ ID NO: 9) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:23.

In an embodiment, the myoglobin is derived from rat myoglobin (SEQ ID NO: 10). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 10. A nucleic acid encoding said myoglobin which is derived from rat myoglobin (SEQ ID NO: 10) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:24.

In an embodiment, the myoglobin is derived from tuna myoglobin (SEQ ID NO: 11). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 11. A nucleic acid encoding said myoglobin which is derived from tuna myoglobin (SEQ ID NO: 11) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:25.

In an embodiment, the myoglobin is derived from salmon myoglobin (SEQ ID NO: 12). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 12. A nucleic acid encoding said myoglobin which is derived from salmon myoglobin (SEQ ID NO: 12) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:26.

In an embodiment, the myoglobin is derived from blue bonito myoglobin (SEQ ID NO: 13). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 13. A nucleic acid encoding said myoglobin which is derived from blue bonito myoglobin (SEQ ID NO: 13) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:27.

In an embodiment, the myoglobin is derived from sword fish myoglobin (SEQ ID NO: 14). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 14. A nucleic acid encoding said myoglobin which is derived from sword fish myoglobin (SEQ ID NO: 14) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:28.

Myoglobins which may be represented by SEQ ID NO: 1-14 may be encoded by nucleic acids which may be represented by SEQ ID NO: 15-28, respectively, although it is understood that these nucleic acids sequence may need to be codon-optimized in order to be expressed by the microorganism or in order to optimize their production by the microorganism. In the context of the invention the myoglobin used may be considered as recombinant protein.

A "recombinant protein" may mean a protein, or a polypeptide encoded by a recombinant DNA, and produced by an expression system (or expression host or a microorganism), wherein said DNA is not endogenous or native for said expression system. DNA regulatory regions as promoters may also be non native for the expression system. Alternatively DNA regulatory regions such as promoters may be native for said expression system. In other words, the expression system has been transformed, introducing exogenous or foreign DNA into it. This expression system hence formed expresses said recombinant DNA that will be translated into a corresponding recombinant protein, during a production process such as a microbial fermentation process.

### 3. Microbial fermentation

In an embodiment, the microbial fermentation comprises the extracellular secretion of the hemeprotein. In an embodiment, the hemeprotein is extracellularly secreted from the microbial host, and/or wherein the hemeprotein is recombinant hemeprotein.

A microorganism used in the microbial fermentation may be a prokaryote, a eukaryote or a filamentous fungus. A prokaryote may be a bacterium. The bacterium may be a Gram positive/Gram negative bacterium slected from the following list: *Absidia, Achromobacter, Acinetobacter, Aeribacillus, Aneurinibacillus, Agrobacterium, Aeromonas, Alcaligenes, Arthrobacter, Arzoarcus, Azomonas, Azospirillum, Azotobacter, Bacillus, Beijerinckia, Bradyrhizobium, Brevibacills, Burkholderia, Byssochlamys, Citrobacter, Clostridium, Comamonas, Cupriavidus, Corynebacterium, Deinococcus, Escherichia, Enterobacter, Flavobacterium, Fusobacterium, Gossypium, Klebsiella, Lactobacillus, Listeria, Megasphaera, Micrococcus, Mycobacterium, Norcadia, Porphyromonas, Propionibacterium, Pseudomonas, Ralstonia, Rhizobium, Rhodopseudomonas, Rhodospirillum, Rodococcus, Roseburia, Shewanella, Streptomycetes, Xanthomonas, Xylella, Yersinia, Treponema, Vibrio, Streptococcus, Lactococcus, Zymomonas, Staphylococcus, Salmonella, Sphingomonas, Sphingobium, Novosphingobium, Brucella* and *Microscilla.* Preferred bacteria include *Aeribacillus pallidus, Aneurinibacillus terranovensis, Bacillus subtilis, Bacillus amyloliquefaciens, Bacillus coagulans, Bacillus licheniformis, Bacillus megaterium, Bacillus halodurans, Bacillus pumilus, Brevibacillus thermoruber, Brevibacillus panacihumi, Cupriavidus basilensis, G. Iraustophilus, Gluconobacter oxydans, Caulobacter crescentus CB 15, Methylobacterium extorquens, Rhodobacter sphaeroides, Pelotomaculum thermopropionicum, Pseudomonas zeaxanthinifaciens, Pseudomonas putida, Paracoccus denitrificans, Escherichia coli, Corynebacterium glutamicum, Staphylococcus carnosus, Streptomyces lividans, Sinorhizobium melioti, Sphingobium sp., Novosphingobium sp., Sphingomonas henshuiensis,* and *Rhizobium radiobacter.* A preferred bacterium is Escherichia coli. Preferred Escherichia coli strains include: 58, 679, WG1, DH5α, TG1, TOP10, K12, BL21, BL21 DE3, XL1-Blue, XL10-Gold, TB1, REG-12, W945, HB101, DH1, DP50, AB284, JC9387, AG1, C600, Cavalli Hfr, Y10.

A eukaryote may be a yeast or a filamentous fungus. Preferred yeasts include *Saccharomyces, Kluyveromyces, Candida, Pichia, Schizosaccharomyces, Hansenula, Kloeckera, Schwanniomyces, Yarrowia, Cryptococcus, Debaromyces, Saccharomycecopsis, Saccharomycodes, Wickerhamia, Debayomyces, Hanseniaspora, Ogataea, Kuraishia, Komagataella, Metschnikowia, Williopsis, Nakazawaea, Torulaspora, Bullera, Rhodotorula, Sporobolomyces.* Within yeasts, the species *Kluyveromyces lactis, Saccharomyces cerevisiae, Hansenula polymorpha* (also known as *Ogataea henricii*), *Yarrowia lipolytica, Candida tropicalis* and *Pichia pastoris* (also known as *Komagataella phaffii*) are preferred. Preferred Pichia strains are selected from the following list: Bg09, Bg10, Bg11, Bg12 (exemplified), Bg20, Bg21, Bg22, Bg23, Bg24, Bg25, Bg26, Bg40, Bg43, Bg44, Bg45, Y-11430, X-33, GS115, KM71, SMD1168, SMD1165, MC100-3, most preferred Bg10 and derivatives. Preferred Saccharomyces strains are selected from the following list: S288C, CEN.PK family, CBS 2354, ATCC 2360, ATCC 4098, ATCC 4124, ATCC 4126, ATCC 4127, ATCC 4921, ATCC 7754, ATCC 9763, ATCC 20598, ATCC 24855, ATCC 24858, ATCC 24860, ATCC 26422, ATCC 46523, ATCC 56069, ATCC 60222, ATCC 60223, ATCC 60493, ATCC 66348, ATCC 66349, ATCC 96581. A preferred yeast is a *Pichia* strain, more preferably *Pichia pastoris.*

A filamentous fungus may be selected from the following list including: *Acremonium, Agaricus, Aspergillus, Aureobasidium, Chrysosporium, Coprinus, Cryptococcus, Filibasidium, Fusarium, Humicola, Magnaporthe, Mucor, Myceliophthora, Neocallinastix, Neurospora, Paecilomyces, Penicillium, Piromyces, Panerochaete, Pleurotus, Schizophyllum, Talaromyces, Thermoascus, Thielavia, Tolypocladium, Ustilago and Trichoderma.* Preferred filamentous fungus are selected from the following list: *Aspergillus niger, Aspergillus nidulans, Aspergillus fumigatus, Aspergillus oryzae, Aspergillus vadensis, Penicillium chrysogenum, Penicillium citrinum, Penicillium rubens, Penicillium oxalicum, Penicillium subrubescens, Rasamsonia emersonii, Talaromyces emersonii, Acremonium chrysogenum, Trichoderma reesei, Aspergillus sojae,* and *Chrysosporium lucknowense.* Preferred strains of filamentous fungus are selected from the following list: *Aspergillus niger* CBS 513.88, N593, CBS 120.49, N402, ATCC 1015 *Aspergillus oryzae* ATCC 20423, IFO 4177, ATCC 1011, ATCC 9576, ATCC 14488-14491, ATCC 11601, ATCC12892, *Aspergillus vadensis* CBS 113365, CBS 102787, IMI 142717, IBT 24658, CBS 113226, *Penicillium chrysogenum* CBS 455.95, *Penicillium citrinum* ATCC 38065, *Penicillium chrysogenum* P2, Wisconsin 54-1255, *Penicillium subrubescens* CBS 132785, FBCC 1632, *Talaromyces emersonii* CBS 393.64, *Acremonium chrysogenum* ATCC 36225 or ATCC 48272, *Trichoderma reesei* ATCC 26921 or ATCC56765 or ATCC 26921, *Aspergillus sojae* ATCC11906, *Chrysosporium lucknowense* ATCC44006. In a preferred embodiment, *Aspergillus* is used as a filamentous fungus. More preferably, an *Aspergillus niger* strain is used.

In an embodiment, the microorganism used in the microbial fermentation may be a bacterium, a yeast, a filamentous fungus or a cultured mammalian cell line, preferably *Escherichia coli* or *Saccharomyces cerevisiae.* In this context, single, isolated, cultured mammalian cells may be considered as microorganisms. In a further embodiment, the microorganism may be a bacterium, a yeast or a filamentous fungus. The microorganisms in the context of this invention are useful for the production of a hemeprotein or a recombinant hemeprotein, preferably a myoglobin, or a recombinant myoglobin. The microbial fermentation comprises culturing the microorganisms in a suitable medium and optionally recovering the microorganism and/or the hemeprotein or the recombinant hemeprotein, preferably the myoglobin, or the recombinant myoglobin. Optionally, the produced hemeprotein or recombinant hemeprotein, preferably the myoglobin or the recombinant myoglobin does not comprise a signal peptide as defined elsewhere herein. In an embodiment, the recovered hemeprotein or the recombinant hemeprotein, preferably the myoglobin or the recombinant myoglobin is purified. Preferably, the purified hemeprotein or the recombinant hemeprotein, preferably the myoglobin or the recombinant myoglobin has a purity of at least 70%, more preferably has a purity of at least 75%, 80%, 85%, 90%, 95%, 96%, 97%, 98%, 99%, most preferably has a purity of 100%.

Cell culturing during the fermentation by said microorganism may be performed for a duration of 14, 13.5, 13, 12.5, 12, 11.5, 11, 10.5, 10, 9.5, 9, 8.5, 8, 7.5, 7, 6.5, 6, 5.5, 5, 4.5, 4, 3.5, 3, 2.5, 2, 1.5 or 1 days, wherein said duration may deviate by 20%. Preferably, cell culturing is performed for a duration of 14, 13.5, 13, 12.5, 12, 11.5, 11, 10.5, 10, 9.5, 9, 8.5, 8, 7.5, 7, 6.5, 6, 5.5, 5, 4.5, 4, 3.5, 3, 2.5, 2, 1.5 or 1 days, wherein said duration may deviate by 10%. More preferably, the cell culturing is performed for 5 days, wherein said duration may deviate by 20%, most preferably by 10%.

In the context of the method for the production of heme, the cell culturing will result in a heme production of at least 105 mg/L, 140 mg/L, 175 mg/L, 210 mg/L, 245 mg/L, 280 mg/L, 315 mg/L or 350 mg/L of heme. In some embodiments, the cell culturing will result in a production of at least 105 mg/L of heme.

In the context of the method for the production of a hemeprotein, the cell culturing will result in a production of at least 3 g/L, 4 g/L, 5 g/L, 6 g/L, 7 g/L, 8 g/L, 9 g/L or 10 g/L of a hemeprotein, preferably a myoglobin. In some embodiments, the cell culturing will result in a production of at least 3 g/L of a hemeprotein, preferably a myoglobin. The microbial production methods for heme or hemeproteins of the prior art typically achieve a product of 1-60 mg/L and 1-1.5 g/L, respectively.

Accordingly, in some embodiments, the method for the production of heme or a hemeprotein is capable of increasing heme or hemeprotein yield at least 1.1, 1.2, 1.3, 1.4, 1.5, 1.6, 1.7, 1.8, 1.9, or 2-fold compared to the methods known in the art. In preferred embodiments, the method is capable of increasing heme or hemeprotein yield at least 1.4, 1.5, 1.6, 1.7, 1.8, 1.9-fold compared to the methods known in the art. In even more preferred embodiments, the method is capable of increasing heme or hemeprotein yield at least 1.6, 1.7, 1.8, 1.9-fold compared to the methods known in the art. In a most preferred embodiment, the method is capable of increasing heme or hemeprotein yield at least 1.9-fold compared to the methods known in the art.

The cell culturing will typically result in at least 1%, 2%, 3%, 4%, 5%, 6%, 7%, 8, 9%, 10%, 20%, 25%, 30%, 35%, 40%, 45%, 50%, 55%, 60%, 65%, 70%, 75%, 80%, preferably at least 1%, more preferably at least 5%, most preferably at least 10% of the carbon source in the growth medium being converted to a hemeprotein, preferably a myoglobin.

Cell culturing may also be performed by implementation of a multiple step, preferably a two-step, culture method. For example, a production step of heme or a hemeprotein such as a myoglobin may be preceded by a cellular biomass growth step, wherein only limited production or no production of a heme or hemeprotein is taking place. The different steps may be carried out using different culture modes and/or different growth media and/or different culture process parameter values, depending on the goal of each step and/or the characteristics of the cultured cell. The biomass during the production step may or may not be actively growing.

The host cells, heme and/or hemeprotein may optionally be recovered from the culture medium. When present intracellularly, the heme or hemeprotein may optionally be recovered from the recovered host cells (cellular biomass). Optionally, the recovered heme or hemeprotein is purified. Preferably, purification of the heme or hemeprotein will result in a purity of at least 70%, more preferably at least 75%, 80%, 85%, 90%, 95%, 96%, 97%, 98%, 99%, most preferably in heme or a hemeprotein that is substantially pure.

In an embodiment, the microorganism produces the heme or hemeprotein, preferably the myoglobin, extracellularly. The heme or hemeprotein is transported out of the host cell after it is synthesized in the host cell. In this context, both secretory and extracellular fermentations are considered to be extracellular productions. Without being bound to this theory, an extracellular production process has the advantage that the downstream processing to recover the produced heme or hemeprotein is more convenient, efficient and/or effective. Furthermore, an extracellular production process may result in a composition comprising the heme or hemeprotein with a high purity such as at least 60%, 65%, 70%, 75%, 80%, 85%, 90%, 95%, 96%, 97%, 98%, or 99% with minimal downstream processing compared to intracellular methods wherein the heme or hemeprotein is not transported out of the host cell after its synthesis. Purity may be measured as the weight percentage of the total protein fraction in the cell-free supernatant obtained at the end of a process or extracellular process according to the invention. Without being bound to this theory, an extracellular production process has the advantage that the optional step of recovering heme or hemeprotein does not comprise lysing the host cell. As a result, the extracellular process may result in a composition having a low concentration of nucleic acids originating from the host cell.

The relevant downstream processing technology that may be suitable for recovery and/or purification will depend on whether the heme or hemeprotein is accumulated within the cultured cells or excreted. Said processing technology and the associated choice will be known to the skilled person and is discussed, for example, in Wesselingh, J.A and Krijgsman, J., 1st edition, Downstream Processing in Biotechnology, Delft Academic Press, NL, 2013. In a recovery process, the biomass may be recovered from the culture medium using e.g., centrifugation or filtration. If the produced heme or hemeprotein is accumulated within the cells, it can then be recovered and/or purified from the biomass. If it is excreted, it can be recovered from the cell-free medium or, if the biomass separation step is skipped, directly from the culture broth. Recovery and/or purification may be performed according to any conventional recovery or purification methodology known in the art. Methods for recovery and/or purification of proteins are known to the skilled person and are discussed in standard handbooks, such as Sambrook and Russel, Molecular Cloning: A Laboratory Manual, 3rd edition, Cold Spring Harbor Laboratory, Cold Spring Harbor Laboratory Press, NY, 2001 or Ausubel F. et al, eds., Current protocols in molecular biology, Green Publishing and Wiley Interscience, NY, 2003. Examples of widely used recovery and/or purification methods include chromatographic methods such as ultrafiltration, microfiltration, gel-filtration chromatography, ion-exchange chromatography, immunoaffinity chromatography, metal affinity chromatography, fractionation with precipitants such as ammonium sulfate and polyethylene glycol, gel electrophoresis and salting out and dialysis. Preferably, metal affinity chromatography or size-exclusion chromatography is used. Recovery and/or purification may optionally be enhanced by linking the enzyme polypeptide to a sequence that facilitates purification, such as with a GST domain, using well-known molecular toolbox techniques. Optionally, the sequence that facilitates purification and/or signal peptide that facilitates excretion of the hemeprotein is removed from the final product using techniques known in the art, for example proteolysis by endopeptidases targeting a linker between the sequence that facilitates purification and/or the signal peptide and the hemeprotein. In some embodiments, the enzyme polypeptide is linked (fused) to a hexa-histidine peptide, such as the tag provided in a pET23a(+) vector (Genescript Biotech, Leiden, The Netherlands), among others, many of which are commercially available. As, for example, described in Gentz et al., Proc. Natl. Acad. Sci. USA 86:821-824 (1989), hexa-histidine peptide provides for convenient purification of the fusion protein.

In a preferred embodiment, the heme or hemeprotein is obtained via recovering and/or purifying from the culture medium. This may be realized continuously with the production process or subsequently to it. In a preferred embodiment, the heme or hemeprotein is obtained via recovering and/or purifying from the cultured cells. A filter may be used for the purification of the recovered hemeprotein. This may be realized continuously with the production process, by harvesting fractions of growing cells, or subsequently to it.

In an embodiment, the heme or hemeprotein is sterilized, shredded, spray dried, spray dried, freeze dried, blended, shaped, cubed, dosed or packed. Sterilization refers to any process that removes, kills, or deactivates all forms of life (particularly microorganisms such as fungi, bacteria, spores, and unicellular eukaryotic organisms) and other biological agents such as prions present in or on a specific surface, object, or fluid. Sterilization can be achieved through various means, including heat, chemicals, irradiation, high pressure, and filtration. Freeze drying, also known as lyophilization or cryodesiccation, is a low temperature dehydration process that involves freezing the product, lowering pressure, then removing the ice by sublimation. Packing aims to provide a protection for the product, to tamper resistance and to provide physical, chemical or biological needs. Packing may also contain nutrition facts, characteristics of the products, and an instruction/guide for use of the product.

In a preferred embodiment, the cultured host cells used in fermentation are immobilized. Immobilization of cells may be achieved by any means known to the skilled person as discussed in standard handbooks such as Guisan, J.M., Bolivar, J.M., López-Gallego, F., Rocha-Martin, J. (Eds.), Immobilization of Enzymes and Cells: Methods and Protocols, Springer US, USA, 2020. Typically, the host cells can be immobilized to a semi-solid or solid support by three different methods. The first method involves polymerizing or solidifying a spore- or cell-containing solution. Examples of polymerizable or solidifiable solutions include alginate, A-carrageenan, chitosan, polyacrylamide, polyacrylamide-hydrazide, agarose, polypropylene, polyethylene glycol, dimethyl acrylate, polystyrene divinyle benzene, polyvinyl benzene, polyvinyl alcohol, epoxy carrier, cellulose, cellulose acetate, photocrosslinkable resin, prepolymers, urethane, and gelatin. The second method involves cell adsorption onto a support. Examples of such supports include bone char, cork, clay, resin, sand porous alumina beads, porous brick, porous silica, celite, or wood chips. The host cells can colonize the support and form a biofilm. The third method involves the covalent coupling of the host cells to a support using chemical agents like glutaraldehyde, o-dianisidine (U.S. Pat. No. 3,983,000), polymeric isocyanates (U.S. Pat. No. 4,071,409), silanes (U.S. Pat. Nos. 3,519,538 and 3,652,761), hydroxyethyl acrylate, transition metal-activated supports, cyanuric chloride, sodium periodate, toluene, and the like. Cultured host cells can be immobilized in any phase of their growth, for example after a desired cell density in the culture has been reached. Suitable culture modes and/or different culture process parameter values will be known to the skilled person and are discussed in standard handbooks, such as Colin R. Phillips C.R., Poon Y. C., Immobilization of Cells: In Biotechnology Monographs book series (Biotechnology, volume 5), Springer, Berlin, Germany, 1988; Tampion J., Tampion M. D., Immobilized Cells: Principles and Applications, Cambridge University Press, UK, 1987. Preferably, immobilized cells are cultured in packed bed bioreactors, also known as plug-flow bioreactors, or expanded (fluidized) bed bioreactors. Suitable growth media and recovery and/or purification methods are further discussed elsewhere herein.

In preferred embodiments, a yeast strain according to the invention comprises one or more heterologous nucleic acid molecules comprising a nucleotide sequence encoding the hemeprotein, preferably the myoglobin as described herein.

In some embodiments, a yeast strain according to the invention comprises at least one, at least two, at least three, at least four, or at least five heterologous nucleic acid molecules comprising a nucleotide sequence encoding the hemeprotein as described herein. In preferred embodiments, a yeast strain according to the invention comprises at least three or at least five heterologous nucleic acid molecules comprising a nucleotide sequence encoding the hemeprotein as described herein. Accordingly, in some embodiments, a yeast strain according to the invention comprises at least three heterologous nucleic acid molecules comprising a nucleotide sequence encoding the hemeprotein as described herein. In some embodiments, a yeast strain according to the invention comprises at least five heterologous nucleic acid molecules comprising a nucleotide sequence encoding the hemeprotein as described herein.

In even more preferred embodiments, a yeast strain according to the invention comprises five or more heterologous nucleic acid molecules comprising a nucleotide sequence encoding the hemeprotein as described herein.

In some embodiments, the heterologous nucleic acid molecule comprising a nucleic acid sequence encoding the hemeprotein as described herein is operably linked to a promoter. Accordingly, in some embodiments, the heterologous nucleic acid molecule as described herein further comprises a promoter. In preferred embodiments, there is provided a heterologous nucleic acid molecule as described herein, wherein the promoter is a constitutive promoter. A description of "promoter" has been provided under the section entitled "general information".

A promoter as used herein encompasses derivatives of promoters and should exert at least an activity of a promoter as known to a person of skill in the art (especially when the promoter sequence is described as having a minimal identity percentage with a given SEQ ID NO). Preferably, a promoter described as having a minimal identity percentage with a given SEQ ID NO should control transcription of the nucleotide sequence to which it is operably linked as assessed in an assay known to a person of skill in the art. For example, such assay could involve measuring expression of the transgene. Expression may be assessed as described under the section entitled "general information" or as shown in the Examples.

If a yeast is used as host cell, the following regulatory regions may be used. A promoter suitable to be used in yeast may be a constitutive promoter. Examples of suitable constitutive promoters include: a glycolytic promoter selected from FBA1, TPI1, PGK1, PYK1, TDH3, ENO2, HXK2, PGI1, PFK1, PFK2, GPM1 gene or a non-glycolytic promoter of the TEF2 gene, or derivatives thereof.

A suitable promoter to be used in yeast may be inducible. If the yeast is a Pichia, the methanol inducible promoter AOX1 is preferred. Otherwise, the GAL1 promoter (galactose-inducible) may be used when the yeast is S. cerevisiae. Derivatives of promoters as described herein comprise promoters that have been mutated as to differentiate the directed expression of the transgenes operably linked to said promoters as compared to the non-mutated promoters, which can be increased or decreased, preferably increased. Methods of mutating nucleotide sequences are known to the skilled person and can comprise any of introduction of single nucleotide polymorphisms, nucleotide insertions and nucleotide deletions.

In preferred embodiments, the heterologous nucleic acid molecule comprising a nucleic acid sequence encoding the hemeprotein may further comprise a sequence encoding a signal peptide. In other words, in the context of this invention, a preferred heterologous nucleic acid molecule comprising a nucleic acid sequence encoding the hemeprotein is one with a signal peptide. The term "signal peptide" is understood herein as a peptide operably linked (fused) in frame to the amino terminus of a polypeptide having biological activity and directing the polypeptide towards one or more cellular or extracellular compartments. In some embodiments, the signal peptide directs the heterologous nucleic acid molecule comprising a nucleic acid sequence encoding the hemeprotein toward the secretory pathway. Accordingly, in some embodiments, the hemeprotein as described herein is transported out of the cell after it is synthesized in the host cell. A preferred signal peptide for excretion for Pichia (and Saccharomyces) includes: the S. cerevisiae alpha mating factor pre- pro- secretion signal peptide, the S. cerevisiae Ost1 signal peptide, the S. cerevisiae Aga2 signal peptide and fusions thereof.

In some embodiments, the hemeprotein as described herein is obtained by, obtainable by, or directly obtained by the method for the production of a hemeprotein as described herein (See section 'Methods and uses'). The terms "obtained by", "obtainable by", or "directly obtained by" may be used interchangeably throughout this application. In some embodiments, the hemeprotein as described herein is obtained by isolating the hemeprotein from a yeast strain as described elsewhere.

### Flavour composition

In the context of the present invention, the hemeproteins "or heme-proteins" as described herein, are not peptides, or hemeprotein derived peptides, or hemeprotein hydrolyzed peptides. A peptide derived from a corresponding full-length protein is a short chain of amino acids that is cleaved or derived from the larger precursor (or full-length or wild type) protein. "Short" may mean the size of the peptide is at least 5%, 10%, 20%, 30%, 40%, 50% the size of the corresponding precursor (or full-length or wild type) protein.

In some embodiments, flavour composition of the present invention comprises at least part of the fermentation broth or part of the microbial host.

In an embodiment, the flavor composition comprising a heme protein as provided by the present invention still comprises at least part of the fermentation broth from the microbial fermentation as described herein or part thereof. "Fermentation broth" refers to the liquid medium or cell culture medium in which microorganisms used in the microbial fermentation are cultured to produce the myoglobin as described herein. Fermentation broth may contain at least part of the microbial host used to produce the myoglobin. Within this context, "par thereof" or "at least part of" may mean at least 20%, 30%, 40%, 50%, 60%, 70%, 80%, 90% of the initial mass amount (of the fermentation broth or of the microbial host) present at the end of the fermentation process. In an embodiment, the (total) weight concentration of the fermentation broth in the flavor composition is at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%. In an embodiment, the weight fraction of myoglobin in the fermentation broch in the flavor composition is at least 5%, 10%, 15%, 20%, 25%, 30%, 35%, 40%, 45%, or 50%.

In an embodiment, the flavor composition comprising a heme protein as provided by the present invention comprises at least part of the microorganisms used in the microbial fermentation as described herein. In an embodiment, the microorganism is not alive or viable. In an embodiment, the microorganism is not intact. In this context, "intact" may mean that the microorganism is lysed, or part of the microorganism is obtained. In an embodiment, the microorganism is yeast. In an embodiment, the (total) weight concentration of the yeast in the flavour composition is at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%. In an embodiment, the weight fraction of hemeprotein in the fermentation yeast in the flavour composition is at least 5%, 10%, 15%, 20%, 25%, 30%, 35%, 40%, 45%, or 50%. In an embodiment, the heme protein is myoglobin. In an embodiment, the yeast is *Pichia pastoris.* In some preferred embodiment, the heme-protein is not a peptide, and has a length of at least 90% of the total number of amino acids in the full length heme-protein. In some preferred embodiment, the myoblogin is not a peptide, and has a length of at least 90% of the total number of amino acids in the fulllength myoglobin, more preferably full length myoglobin derived from any one of SEQ ID NO:1-14 as described herein.

In an embodiment, the flavor composition comprising a heme protein as provided by the present invention comprises yeast extract, wherein the yeast is used as the microorganisms in the microbial fermentation. In an embodiment, the (total) weight concentration of the yeast extract in the flavour composition is at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%. A "yeast extract" refers to yeast cell contents without cell walls. Typically, yeast extracts are produced from steps of: fermentation (growing the yeast), disruption (breaking of the cells), and separation (to keep the soluble part of the yeast cellular content). In an embodiment, the weight fraction of hemeprotein in the yeast extract in the flavour composition is at least 5%, 10%, 15%, 20%, 25%, 30%, 35%, 40%, 45%, or 50%. In an embodiment, the heme protein is myoglobin. In an embodiment, the yeast is *Pichia pastoris,* and the yeast extract is *Pichia pastoris* extract. In some preferred embodiment, the heme-protein is not a peptide, and has a length of at least 90% of the total number of amino acids in the full length heme-protein. In some preferred embodiment, the myoblogin is not a peptide, and has a length of at least 90% of the total number of amino acids in the full length myoglobin, more preferably full length myoglobin derived from any one of SEQ ID NO:1-14 as described herein.

All these previously defined embodiments comprising either the fermentation broth, the microorganism host, the culture medium and/or yeast extract are advantageous. Flavour compositions comprising any of these elements in parallel to the myoglobin defined herein are expected to exhibit improved properties in terms of amount of bioavailable source of iron and/or in terms of amounts of essential nutrients.

In an embodiment, the (total) weight concentration of hemeproteins in the flavour composition is at least 0.005%, 0.01%, 0.015%, 0.02%, 0.025%, 0.03%, 0.035%, 0.04%, 0.045%, 0.05%, 0.055%, 0.06%, 0.065%, 0.07%, 0.075%, 0.08%, 0.085%, 0.09%, 0.095%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 6%, 7%, 8%, 9%, 10%, preferably lower than 10%, more preferably lower than 5%. In some preferred embodiment, the heme-protein is not a peptide, and has a length of at least 90% of the total number of amino acids in the full length heme-protein. In an embodiment, the (total) weight concentration of myoglobins in the flavour composition is at least 0.005%, 0.01%, 0.015%, 0.02%, 0.025%, 0.03%, 0.035%, 0.04%, 0.045%, 0.05%, 0.055%, 0.06%, 0.065%, 0.07%, 0.075%, 0.08%, 0.085%, 0.09%, 0.095%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 6%, 7%, 8%, 9%, 10%, preferably lower than 10%, more preferably lower than 5%. In some preferred embodiment, the myoblogin is not a peptide, and has a length of at least 90% of the total number of amino acids in the full length myoglobin, more preferably full length myoglobin derived from any one of SEQ ID NO:1-14 as described herein.

In an embodiment, the weight fraction of myoglobins in the heme-proteins in the flavour composition is at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%. In some preferred embodiment, the heme-protein is not a peptide, and has a length of at least 90% of the total number of amino acids in the full length heme-protein. In some preferred embodiment, the myoblogin is not a peptide, and has a length of at least 90% of the total number of amino acids in the full length myoglobin, more preferably full length myoglobin derived from any one of SEQ ID NO:1-14 as described herein.

In an embodiment, the (total) weight concentration of hemeproteins in the flavour composition is at least 0.005%, 0.01%, 0.015%, 0.02%, 0.025%, 0.03%, 0.035%, 0.04%, 0.045%, 0.05%, 0.055%, 0.06%, 0.065%, 0.07%, 0.075%, 0.08%, 0.085%, 0.09%, 0.095%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 6%, 7%, 8%, 9%, 10%, and the weight fraction of myoglobins in the hemeproteins in the flavour composition is at least 90%. In some preferred embodiment, the heme-protein is not a peptide, and has a length of at least 90% of the total number of amino acids in the full length heme-protein . In some preferred embodiment, the myoblogin is not a peptide, and has a length of at least 90% of the total number of amino acids in the full length myoglobin, more preferably full length myoglobin derived from any one of SEQ ID NO:1-14 as described herein.

In an embodiment, the (total) weight concentration of hemeproteins in the flavour composition is at least 0.05%, and the weight fraction of myoglobins in the hemeproteins in the flavour composition is at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%. In some preferred embodiment, the heme-protein is not a peptide, and has a length of at least 90% of the total number of amino acids in the full length heme-protein. In some preferred embodiment, the myoblogin is not a peptide, and has a length of at least 90% of the total number of amino acids in the full length myoglobin, more preferably full length myoglobin derived from any one of SEQ ID NO:1-14 as described herein.

In an embodiment, the (total) weight concentration of heme-proteins in the flavour composition is at least 0.1%, and the weight fraction of myoglobins in the heme-proteins in the flavour composition is at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%. In some preferred embodiment, the heme-protein is not a peptide, and has a length of at least 90% of the total number of amino acids in the full length heme-protein. In some preferred embodiment, the myoblogin is not a peptide, and has a length of at least 90% of the total number of amino acids in the full length myoglobin, more preferably full length myoglobin derived from any one of SEQ ID NO:1-14 as described herein.

In an embodiment, the (total) weight concentration of hemeproteins in the flavour composition is at least 0.5%, and the weight fraction of myoglobins in the heme-proteins in the flavour composition is at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%. In some preferred embodiment, the heme-protein is not a peptide, and has a length of at least 90% of the total number of amino acids in the full length heme-protein. In some preferred embodiment, the myoblogin is not a peptide, and has a length of at least 90% of the total number of amino acids in the full length myoglobin, more preferably full length myoglobin derived from any one of SEQ ID NO:1-14 as described herein.

In an embodiment, the (total) weight concentration of hemeproteins in the flavour composition is at least 1.0%, and the weight fraction of myoglobins in the heme-proteins in the flavour composition is at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%. In some preferred embodiment, the heme-protein is not a peptide, and has a length of at least 90% of the total number of amino acids in the full length heme-protein. In some preferred embodiment, the myoblogin is not a peptide, and has a length of at least 90% of the total number of amino acids in the full length myoglobin, more preferably full length myoglobin derived from any one of SEQ ID NO:1-14 as described herein.

In an embodiment, the (total) weight concentration of heme-proteins in the flavour composition is at least 3.0%, and the weight fraction of myoglobins in the heme-proteins in the flavour composition is at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%. In some preferred embodiment, the heme-protein is not a peptide, and has a length of at least 90% of the total number of amino acids in the full length heme-protein. In some preferred embodiment, the myoblogin is not a peptide, and has a length of at least 90% of the total number of amino acids in the full length myoglobin, more preferably full length myoglobin derived from any one of SEQ ID NO:1-14 as described herein.

In an embodiment, at least 50%, 55%, 60%, 65%, 70%, 75%, 80%, 85%, 90%, or 95% of heme-proteins in the flavour composition originate from the addition of the hemeproteins (per weight). In a more preferred embodiment, at least 50%, 55%, 60%, 65%, 70%, 75%, 80%, 85%, 90%, or 95% of myoglobins in the flavour composition are recombinant myoglobin (per weight). In some preferred embodiment, the heme-protein is not a peptide, and has a length of at least 90% of the total number of amino acids in the full length heme-protein. In some preferred embodiment, the myoblogin is not a peptide, and has a length of at least 90% of the total number of amino acids in the full length myoglobin, more preferably full length myoglobin derived from any one of SEQ ID NO:1-14 as described herein.

In an embodiment, at least 50%, 55%, 60%, 65%, 70%, 75%, 80%, 85%, 90%, or 95% of myoglobins in the flavour composition originate from the addition of the myoglobin (per weight). In a more preferred embodiment, at least 50%, 55%, 60%, 65%, 70%, 75%, 80%, 85%, 90%, or 95% of myoglobins in the flavour composition are recombinant myoglobin (per weight). In some preferred embodiment, the myoblogin is not a peptide, and has a length of at least 90% of the total number of amino acids in the full length myoglobin, more preferably full length myoglobin derived from any one of SEQ ID NO:1-14 as described herein.

In an embodiment, the flavor composition of the present invention comprises iron in the form of hemeprotein-iron complex, prerably myglobin-iron complex. Myoglobin is a relatively small globular protein of about 17 kD, found in heart and skeletal muscles. It carries a single heme group with an atom of iron capable of reversible oxygen binding allowing myoglobin to transport oxygen from the cell surface to mitochondria.

In an embodiment, the flavor composition of the present invention comprises 0.01 mg to 40 mg, 0.01 mg to 39 mg, 0.01 mg to 38 mg, 0.01 mg to 37 mg, 0.01 mg to 36 mg, 0.01 mg to 35 mg, 0.01 mg to 34 mg, 0.01 mg to 33 mg, 0.01 mg to 32 mg, 0.01 mg to 31 mg, 0.01 mg to 30 mg, 0.01 mg to 29 mg, 0.01 mg to 28 mg, 0.01 mg to 27 mg, 0.01 mg to 26 mg, 0.01 mg to 25 mg, 0.01 mg to 24 mg, 0.01 mg to 23 mg, 0.01 mg to 22 mg, 0.01 mg to 21 mg, 0.01 mg to 20 mg, 0.01 mg to 19 mg, 0.01 mg to 18 mg, 0.05 mg to 18 mg, 0.1 mg to 18 mg, 0.2 mg to 18 mg, 0.5 mg to 18 mg, 1 mg to 18 mg, 2 mg to 18 mg, 3 mg to 18 mg, 4 mg to 18 mg, 5 mg to 18 mg, 6 mg to 18 mg, 7 mg to 18 mg, 8 mg to 18 mg, 9 mg to 18 mg, 10 mg to 18 mg, 11 mg to 18 mg, 12 mg to 18 mg, 13 mg to 18 mg, 14 mg to 18 mg, or 15 mg to 18 mg of iron expressed as myoglobin per 100 gram. In this context, the term "iron" may be used interchangeably with the term "Fe". "Expressed as myoglobin" in this context means that the iron or Fe content is sourced from the myoglobin as described herein. In other word, the iron or Fe is contained within the myoglobin comprised in the flavour composition of the present invention. "Sourced from" may mean that the iron or Fe is complexed to or bound to the myoglobin comprised in the flavour composition of the present invention as described herein. In an embodiment, the flavour composition of the present invention comprises 0.01 mg to 18 mg of iron expressed as myoglobin. In some preferred embodiment, the myoblogin is not a peptide, and has a length of at least 90% of the total number of amino acids in the full length myoglobin, more preferably full length myoglobin derived from any one of SEQ ID NO:1-14 as described herein.

In an embodiment, the flavour composition comprises a bioavailable iron or heme iron in an amount ranging from 0.3 to 20 mg bioavailable iron or heme iron per 100 gram of said flavour composition. In an embodiment such amount is ranged from 0.1 mg up to 16.5 mg, 16.17 mg, 15.84 mg, 15.51 mg, 15.18 mg, 14.85 mg, 14.52 mg, 14.19 mg, 13.86 mg, 13.53 mg, 13.2 mg, 12.87 mg, 12.54 mg, 12.21 mg, 11.88 mg, 11.55 mg, 11.22 mg, 10.89 mg, 10.56 mg, 10.23 mg, 9.9 mg, 9.735 mg, 9.57 mg, 9.405 mg, 9.24 mg, 9.075 mg, 8.91 mg, 8.745 mg, 8.58 mg, 8.415 mg, 8.25 mg, 8.085 mg, 7.92 mg, 7.755 mg, 7.59 mg, 7.425 mg, 7.26 mg, 7.095 mg, 6.93 mg, 6.765 mg, 6.6 mg, 6.435 mg, 6.27 mg, 6.105 mg, 5.94 mg, 5.775 mg, 5.61 mg, 5.445 mg, 5.28 mg, 5.115 mg, 4.95 mg, 4.785 mg, 4.62 mg, 4.455 mg, 4.29 mg, 4.125 mg, 3.96 mg, 3.795 mg, 3.63 mg, 3.465 mg, 3.3 mg, 3.135 mg, 2.97 mg, 2.805 mg, 2.64 mg, 2.475 mg, 2.31 mg, 2.145 mg, 1.98 mg, 1.815 mg or 1.65 mg bioavailable iron or heme iron per 100 gram flavour composition. In another embodiment such amount is 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.1, 10.2, 10.3, 10.4, 10.5, 10.6, 10.7, 10.8, 10.9, 11, 11.1, 11.2, 11.3, 11.4, 11.5, 11.6, 11.7, 11.8, 11.9, 12, 12.1, 12.2, 12.3, 12.4, 12.5, 12.6, 12.7, 12.8, 12.9, 13, 13.1, 13.2, 13.3, 13.4, 13.5, 13.6, 13.7, 13.8, 13.9, 14, 14.1, 14.2, 14.3, 14.4, 14.5, 14.6, 14.7, 14.8, 14.9, 15, 15.1, 15.2, 15.3, 15.4, 15.5, 15.6, 15.7, 15.8, 15.9, 16, 16.1, 16.2, 16.3, 16.4, 16.5, 16.6, 16.7, 16.8, 16.9, 17, 17.1, 17.2, 17.3, 17.4, 17.5, 17.6, 17.7, 17.8, 17.9, 18, 18.1, 18.2, 18.3, 18.4, 18.5, 18.6, 18.7, 18.8, 18.9, 19, 19.1, 19.2, 19.3, 19.4, 19.5, 19.6, 19.7, 19.8, 19.9, or 20 mg bioavailable iron or heme iron per 100 gram. In some preferred embodiment, the heme-protein is not a peptide, and has a length of at least 90% of the total number of amino acids in the full length heme-protein. In some preferred embodiment, the myoblogin is not a peptide, and has a length of at least 90% of the total number of amino acids in the full length myoglobin, more preferably full length myoglobin derived from any one of SEQ ID NO:1-14 as described herein.

In an embodiment, the flavor composition of the present invention comprises at least 0.01 % by weight of the hemeprotein as described herein. In an embodiment, the flavor composition of the present invention comprises from 0.01% up to 5%, 4.9%, 4.8%, 4.7%, 4.6%, 4.5%, 4.4%, 4.3%, 4.2%, 4.1%, 4%, 3.9%, 3.8%, 3.7%, 3.6%, 3.5%, 3.4%, 3.3%, 3.2%, 3.1%, 3%, 2.95%, 2.9%, 2.85%, 2.8%, 2.75%, 2.7%, 2.65%, 2.6%, 2.55%, 2.5%, 2.45%, 2.4%, 2.35%, 2.3%, 2.25%, 2.2%, 2.15%, 2.1%, 2.05%, 2%, 1.95%, 1.9%, 1.85%, 1.8%, 1.75%, 1.7%, 1.65%, 1.6%, 1.55%, 1.5%, 1.45%, 1.4%, 1.35%, 1.3%, 1.25%, 1.2%, 1.15%, 1.1%, 1.05%, 1%, 0.95%, 0.9%, 0.85%, 0.8%, 0.75%, 0.7%, 0.65%, 0.6%, 0.55%, 0.5%, 0.4%, 0.3%, 0.2%, or 0.1% by weight of the hemeprotein as described herein. In an embodiment, the hemeprotein is myoglobin as described herein. In some preferred embodiment, the heme-protein is not a peptide, and has a length of at least 90% of the total number of amino acids in the full length heme-protein.

In an embodiment, the flavor composition may be formulated as dry composition, a semi-moist composition, a wet composition, an oil based (or oil soluble or oil dispersible) composition or any combination of these forms. A flavor composition may be formulated as a liquid, solid, or semi-solid flavor composition. By "dry flavor composition" is meant one that has a moisture content less than 15 percent by weight. By "semi-moist flavor composition" is meant one that has a moisture content between 55 to 65 percent by weight. By " wet type flavor composition " is meant one that has a moisture content between 65 to 85 percent by weight. By "oil-based composition" is meant one that can be dissolved in oil-based solutions. Some non-limiting examples of such oil-based composition include salad dressings, marinades, and sauces.

Hereinafter, all percentages referred to are understood to be by weight unless specified otherwise and are based upon the weight of the final flavor composition product.

In an embodiment, the flavour composition is expected to mimick the flavor, color and/or aroma of meat without having all its drawbacks. In an embodiment, the flavour composition is expected to mimick the flavor and/or aroma of meat without having all its drawbacks. In an embodiment, the flavour composition is expected to mimick the flavor of meat without having all its drawbacks. In an embodiment, the flavour composition is expected to mimick the aroma of meat without having all its drawbacks. In the embodiments of this context, this is mostly due to the presence of the myoglobin as described herein. Alternatively, it might have an aspect, form, structure, texture, color, palatability, flavor, aroma and/or appearance which is distinct from the one of meat.

Within the context of the invention, the meat flavor and/or arom experience may be mimicked when the flavour composition of the present invention generates an aroma and/or a flavor compound recognizable by a subject, preferably a human subject, and characteristic of some odorants and/or flavor compound released when meat is cooked. For example, the formation of some of these odorants may be catalyzed by the iron of heme present in the myoglobin. Without being bound to this theory, the formation may be due to lipid oxidation and/or Maillard reactions ("Maillard products"). This situation may be mimicked with the myoglobin within the flavour composition of the invention.

In an embodiment of the invention, the flavour composition is expected to mimick the bloody and/or metallic aroma of meat. Preferably, the flavour composition is able to mimick the bloody and/or metallic aroma of meat to a higher degree than other flavor compositions (i.e. not according to the invention). In the context of this invention, "raw" means not subjected to a heat treatment, preferably uncooked and ungrilled. The metallic aroma in raw meat may be attributed to a variety of volatile organic compounds (VOCs) that are produced during the degradation of proteins or arise from other metabolic processes. Non-limiting examples of VOCs may be aldehydes, ketones, sulfur compounds, esters, or hydrocarbons, The VOCs may be assessed using techniques known to a skilled person, such as Gas Chromatography-Mass Spectrometry (GC-MS), and Solid-Phase Microextraction (SPME) Coupled with GC-MS. The metallic aroma in raw meat may also be assessed using sensory evaluation by trained panels or consumer testing, alone or in combination with the above mentioned techniques.

In an embodiment of the invention, the flavour composition is expected to mimick the roasted aroma of meat, particularly of grilled meat. Preferably, the meat substitute is able to mimick the roasted aroma of meat to a higher degree than other flavour compostion (i.e. not according to the invention). In the context of this invention, "cooked" means subjected to a heat treatment, preferably sterilized, grilled or roasted.

In the context of this invention, and specifically in the embodiments in this section, an "other flavour composition" or "a flavour composition not according to the invention" is preferably a plant-based flavour composition comprising a soy leghemoglobin (LegH).

In an embodiment, the flavour composition has an aroma characterized by a higher concentration of Mailard compounds, i.e. compounds formed due to the Maillard reaction, when cooked, compared to a corresponding flavour composition which does not comprise the myoglobin as described herein.

In an embodiment, the flavour composition has an aroma characterized by a higher concentration of oxidized lipids, lipid oxidation products, pyrazines, pyrroles, aldehydes and/or ketones in the volatile compounds when grilled relative to other flavour composition (i.e. not according to the invention, or other positive control). Higher preferably means at least 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, or 300%. Preferred pyrazines are methylpyrazine, 2,6-dimethylpyrazine, 2,5-dimethylpyrazine, and 2-ethyl-6-methylpyrazine. A preferred pyrrole is pyrrole. Preferred lipid oxidation products are 2-methylbutanal and 3-methylbutanal. Preferred aldehydes are methional, nonanal and octanal. A preferred ketone is 2-acetylthiazole. It is understood that the categories of compounds mentioned in this paragraph are not mutually exclusive, e.g. lipid oxidation products may be aldehydes or ketones.

In the context of the present invention, the "other flavour composition not according to the invention" or "a positive control" may be a yeast extract (not enriched in heme or hemeproteins, made from proprietary yeast strains), combined with salts and sugars, or hydrolysed vegetable proteins, or proteolytic extract from animal meat.

Aromas from flavour composition containing different concentrations of myoglobin may be analyzed using gas chromatography-mass spectrometry (GC-MS) with headspace solid-phase microextraction (HS-SPME).

In an embodiment, the flavour composition of the present invention may be expected to allow a meat experience when the flavour composition provides a similar color with the color of meat. The color of meat is determined by the concentration of heme-containing protein and/or by its oxidation state in the meat. Therefore, the total amount of the myoglobin added to the flavour composition will determine the color of the flavour composition.

In an embodiment, the flavour composition according to the present invention, may comprise a carrier acceptable for food, and/or one or more additives.

In an embodiment, the flavour composition according to the present invention is formulated as edible, preferably comprises an edible carrier. In an embodiment, the flavour composition according to the present invention is formulated for oral administration. In preparing the composition in edible form, or optionally oral dosage form for oral administration, any of the usual media may be utilized. For liquid preparations (e.g., suspensions, elixirs, and solutions), media containing, for example water, oils, alcohols, flavouring agents, preservatives, colouring agents and the like may be used.

In the context of the present invention, the flavour composition is mainly intended to provide flavors to a diet and may not be used to replace whole foods or diet. Accordingly, in the context of the present invention, the flavor composition is not a food product or part thereof. In some embodiments, the flavor composition is not a functional food or food product.

In an embodiment, the flavour composition of the present invention may further comprise multiple vitamins, minerals and/or nutrients. Non limiting examples of said multiple vitamins, minerals and/or nutrients include vitamin A, beta-carotene alone, carotenoids, lycopene, lutein, zeaxanthin, cryptoxanthin, thiamine (vitamin B1), riboflavin (vitamin B2), niacin (vitamin B3), pantothenic acid (vitamin B5), pyridoxine (vitamin B1), folate (vitamin B9), cyanocobalamin (vitamin B12), vitamin C-complex, vitamin D, vitamin E, tocopherols, tocotrieneols, iodine, iron, zinc, copper, magnesium, and/or omega 3 fatty acids.

In some embodiments, said vitamins (i.e. vitamin B), minerals, and/or nutrients are sourced from the fermentation broth and comprised in the flavour composition as described herein. In some embodiments, said vitamins (i.e. vitamin B), minerals, and/or nutrients are sourced from the microbial culture medium, preferably yeast culture medium and comprised in the flavour composition as described herein. In some embodiments, said vitamins (i.e. vitamin B), minerals, and/or nutrients are sourced from the yeast extract and comprised in the flavour composition as described herein.

In an embodiment, the flavour composition of the present invention may further comprise fat. Non limiting examples fat sources may be peanut butter, almonds, cashews, hazelnuts, peanuts, pistachios, rapeseed oil, olive oil, sunflower oil, corn oil, olives, avocados, oily fish like kippers, mackerel and salmon, processed and fatty meats like sausages, ham, burgers and bacon, hard cheeses like cheddar, whole milk, cream, butter, lard, ghee, suet, palm oil, coconut oil, or hard margarines made with hydrogenated oil.

In an embodiment, the flavour composition of the present invention may be formulated as solid or liquid form. In an embodiment, the flavour composition of the present invention may be formulated as a capsule, tablet, caplet, gel caplet (gelcap), syrup, a liquid composition, a frozen liquid composition, a liquid solution, a liquid suspension, a powder, a concentrated powder, a concentrated powder admixed with a liquid, a freeze-dried powder, a spray dried powder, a dissolvable form, or a granulated form. In an embodiment, the flavour composition according to the present invention is formulated as a powder, a capsule, a tablet, or a liquid solution or suspension.

In an embodiment, the flavour composition according to the present invention further comprises one or more food-grade additives selected from the group comprising: mixing aids, emulsifiers, sweeteners, preservatives, colorants, and flavour additives. In an embodiment, the flavour composition according to the present invention may comprise binders. In an embodiment, the flavour composition according to the present invention may comprise diluents. In an embodiment, the flavour composition according to the present invention may comprise lubricants. In an embodiment, the flavour composition according to the present invention may comprise Glidants.

The flavour composition of the present invention may be sugar coated or enteric coated by standard techniques. The unit dose forms may be individually wrapped, packaged as multiple units without limitation. The flavour composition of the present invention may be packaged in unit dose, rolls, bulk bottles, blister packs and combinations thereof, without limitation.

### 4. Foodstuff and beverage

In a second aspect of the present invention, there is provided a use of the flavour composition as described herein for improving the flavour of a foodstuff or a beverage.

Food or foodstuff may be any substance consumed to provide nutritional support and energy to an organism. It can be raw, processed, or formulated and is consumed orally by a subject, preferably a human subject for growth, health, or pleasure. Typical examples of food may be, but not limited to, vegetables, fruits, cooked meat, milk, eggs, mushrooms and seaweed, cereals, corn, wheat, rice, fermented food such as bread, wine, cheese, and yoghurt.

A beverage is a liquid intended for human consumption. Non limiting examples of a beverage include plain drinking water, milk, juice, smoothies, soft drinks, coffee, tea, soup, hot chocolate, wine, cocktails, cider, and beer.

The flavour composition of the present invention may be used for modifying the flavour of a foodstuff or a beverage. The modification of the flavour may result in one or more of the following:
- mimick of the flavor, color and/or aroma of meat or meat product
- mimick of meat flavor and/or aroma
- mimick of a bloody and/metallic aroma of meat
- mimick of a roasted, particularly of a grilled meat.

Some examples of a flavour composition include, but not limited to, a bouillon, a broth, a seasoning, and a flavouring. A broth, or a bouillon is a savory liquid made of water in which meat, fish, or vegetables have been simmered for a short period of time. It is most commonly used to prepare other dishes, such as soups, gravies, and sauces. Commercially prepared liquid broths are available, typically chicken, beef, fish, and vegetable varieties. Dehydrated broth in the form of bouillon cubes were commercialized beginning in the early 20th century. Seasoning is the process of supplementing food via herbs, spices, salts, and/or sugar, intended to enhance a particular flavour.

In an embodiment, the foodstuff is not a meat or a meat analogue. In an embodiment, the beverage does not contain ingredients derived from a meat or a meat analogue.

In an embodiment, the flavour composition or the flavour composition for use as described herein is a bouillon.

A non-limiting example of a flavour composition according to the present invention may be a bouillon comprising the following ingredients:

| **Ingredients** | **Percentage % by weight** |
|---|---|
| Yeast extract (myoglobin of the present invention) | 20% |
| | (5%) |
| salt | 42% |
| hardened vegetable fat | 1% |
| hydrolysed vegetable proteins | 5% |
| starch | 12% |
| herbs | 2% |
| spices | 1% |
| flavourings | 2% |
| sugar | 15% |

### 5. Method for improving the flavour of a foodstuff or a beverage

In a third aspect, there is provided a method for improving the flavour of a foodstuff or a beverage, comprising adding to said foodstuff or beverage one or more flavour compositions as defined herein.

In some embodiments, the one or more flavour compositions comprises one or more heme protein, preferably myoglobin as described herein. In some embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with any one of the SEQ ID NOs: 1-14. In an embodiment, the myoglobin is represented by a sequence having at least 95% identity with any one of SEQ ID NO: 1 to 14.

In an embodiment, the myoglobin is steppe mammoth myoglobin (SEQ ID NO: 1). In an embodiment, the myoglobin is woolly mammoth myoglobin (SEQ ID NO: 2). In an embodiment, the myoglobin is pig myoglobin (SEQ ID NO: 3). In an embodiment, the myoglobin is sheep myoglobin (SEQ ID NO: 4). In an embodiment, the myoglobin is cow myoglobin (SEQ ID NO: 5). In an embodiment, the myoglobin is chicken myoglobin (SEQ ID NO: 6). In an embodiment, the myoglobin is rabbit myoglobin (SEQ ID NO: 7). In an embodiment, the myoglobin is bovine myoglobin (SEQ ID NO: 8). In an embodiment, the myoglobin is mouse myoglobin (SEQ ID NO: 9). In an embodiment, the myoglobin is rat myoglobin (SEQ ID NO: 10). In an embodiment, the myoglobin is tuna myoglobin (SEQ ID NO: 11). In an embodiment, the myoglobin is salmon myoglobin (SEQ ID NO: 12). In an embodiment, the myoglobin is blue bonito myoglobin (SEQ ID NO: 13). In an embodiment, the myoglobin is sword fish myoglobin (SEQ ID NO: 14).

### 6. A method for preparing a flavour composition

In a fourth aspect, there is provided a method for preparing a flavour composition as described herein, wherein the hemeprotein is a hemeprotein obtained from fermentation by a microorganism, and/or wherein the hemeprotein is recombinant hemeprotein. In an embodiment, the hemeprotein is a hemeprotein obtained from fermentation by a microorganism which has been genetically modified to express the hemeprotein. These nucleic acids sequence may need to be codon-optimized in order to be expressed by the microorganism.

In an embodiment, said method uses a microorganism as described in the first aspect of the present invention. In an embodiment, the microorganism is a bacterium, a yeast, or a filamentous fungus, preferably *Pichia pastoris.*

In an embodiment, the encoded or expressed hemeprotein is a myoglobin.

In an embodiment, the encoded or expressed myoglobin is derived from steppe mammoth myoglobin (SEQ ID NO: 1). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 1. A nucleic acid encoding said myoglobin which is derived from steppe mammoth myoglobin (SEQ ID NO:1) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:15.

In an embodiment, the encoded or expressed myoglobin is derived from woolly mammoth myoglobin (SEQ ID NO: 2). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 2. A nucleic acid encoding said myoglobin which is derived from woolly mammoth myoglobin (SEQ ID NO: 2) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:16.

In an embodiment, the encoded or expressed myoglobin is derived from pig myoglobin (SEQ ID NO: 3). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 3. A nucleic acid encoding said myoglobin which is derived from pig myoglobin (SEQ ID NO: 3) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:17.

In an embodiment, the encoded or expressed myoglobin is derived from Sheep myoglobin (SEQ ID NO: 4). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 4. A nucleic acid encoding said myoglobin which is derived from Sheep myoglobin (SEQ ID NO: 4) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:18.

In an embodiment, the encoded or expressed myoglobin is derived from cow myoglobin (SEQ ID NO: 5). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 5. A nucleic acid encoding said myoglobin which is derived from cow myoglobin (SEQ ID NO: 5) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:19.

In an embodiment, the encoded or expressed myoglobin is derived from chicken myoglobin (SEQ ID NO: 6). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 6. A nucleic acid encoding said myoglobin which is derived from chicken myoglobin (SEQ ID NO: 6) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:20.

In an embodiment, the encoded or expressed myoglobin is derived from rabbit myoglobin (SEQ ID NO: 7). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 7. A nucleic acid encoding said myoglobin which is derived from rabbit myoglobin (SEQ ID NO: 7) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:21.

In an embodiment, the encoded or expressed myoglobin is derived from bovine myoglobin (SEQ ID NO: 8). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 8. A nucleic acid encoding said myoglobin which is derived from bovine myoglobin (SEQ ID NO: 8) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:22.

In an embodiment, the encoded or expressed myoglobin is derived from mouse myoglobin (SEQ ID NO: 9). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 9. A nucleic acid encoding said myoglobin which is derived from mouse myoglobin (SEQ ID NO: 9) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:23.

In an embodiment, the encoded or expressed myoglobin is derived from rat myoglobin (SEQ ID NO: 10). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 10. A nucleic acid encoding said myoglobin which is derived from rat myoglobin (SEQ ID NO: 10) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:24.

In an embodiment, the encoded or expressed myoglobin is derived from tuna myoglobin (SEQ ID NO: 11). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 11. A nucleic acid encoding said myoglobin which is derived from tuna myoglobin (SEQ ID NO: 11) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:25.

In an embodiment, the encoded or expressed myoglobin is derived from salmon myoglobin (SEQ ID NO: 12). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 12. A nucleic acid encoding said myoglobin which is derived from salmon myoglobin (SEQ ID NO: 12) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:26.

In an embodiment, the encoded or expressed myoglobin is derived from blue bonito myoglobin (SEQ ID NO: 13). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 13. A nucleic acid encoding said myoglobin which is derived from blue bonito myoglobin (SEQ ID NO: 13) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:27.

In an embodiment, the encoded or expressed myoglobin is derived from sword fish myoglobin (SEQ ID NO: 14). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 14. A nucleic acid encoding said myoglobin which is derived from sword fish myoglobin (SEQ ID NO: 14) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:28.

Cell culturing during the fermentation by said microorganism may be performed for a duration of 14, 13.5, 13, 12.5, 12, 11.5, 11, 10.5, 10, 9.5, 9, 8.5, 8, 7.5, 7, 6.5, 6, 5.5, 5, 4.5, 4, 3.5, 3, 2.5, 2, 1.5 or 1 days, wherein said duration may deviate by 20%. Preferably, cell culturing is performed for a duration of 14, 13.5, 13, 12.5, 12, 11.5, 11, 10.5, 10, 9.5, 9, 8.5, 8, 7.5, 7, 6.5, 6, 5.5, 5, 4.5, 4, 3.5, 3, 2.5, 2, 1.5 or 1 days, wherein said duration may deviate by 10%. More preferably, the cell culturing is performed for 5 days, wherein said duration may deviate by 20%, most preferably by 10%.

The cell culturing will typically result in a production of at least 100 mg/L, 200 mg/L, 300 mg/L, 400 mg/L, 500 mg/L, 600 mg/L, 700 mg/L, 800 mg/L, 900 mg/L, 1 g/L, 2 g/L, 3 g/L, 4 g/L, 5 g/L, 6 g/L, 7 g/L, 8 g/L, 9 g/L, 10 g/L, 11 g/L, 12 g/L, 13 g/L, 14 g/L, 15 g/L, 16 g/L, 17 g/L,18 g/L, 19 g/L, 20 g/L, 21 g/L, 22 g/L, 23 g/L, 24 g/L, 25 g/L, 50 g/L, 75 g/L, 100 g/L, 200 g/L or 300 g/L of a myoglobin.

The cell culturing will typically result in at least 10%, 20%, 25%, 30%, 35%, 40%, 45%, 50%, 55%, 60%, 65%, 70%, 75%, 80%, 85%, 90%, 95%, or 100%, preferably at least 20%, more preferably at least 40%, most preferably at least 60% of the carbon source in the growth medium being converted to a myoglobin.

Cell culturing may also be performed by implementation of a multiple step, preferably a two-step, culture method. For example, a production step of a myoglobin may be preceded by a cellular biomass growth step, wherein only limited production or no production is taking place. The different steps may be carried out using different culture modes and/or different growth media and/or different culture process parameter values, depending on the goal of each step and/or the cultured cell. The biomass during the production step may or may not be actively growing.

The cell culturing step, recovering step and/or purification step described in the first aspect of the present invention can also be used in this fourth aspect of the invention.

### 7. General terms

All patent applications, patents, and printed publications cited herein are incorporated herein by reference in the entireties, except for any definitions, subject matter disclaimers or disavowals, and except to the extent that the incorporated material is inconsistent with the express disclosure herein, in which case the language in this disclosure controls.

One skilled in the art will recognize many methods and materials similar or equivalent to those described herein, which could be used in the practice of the present invention. Indeed, the present invention is in no way limited to the methods and materials described.

The present invention is further described by the following examples which should not be construed as limiting the scope of the invention. The generalization of certain aspects and features disclosed in the below examples to the foregoing description is part of this disclosure.

Unless stated otherwise, all technical and scientific terms used herein have the same meaning as customarily and ordinarily understood by a person of ordinary skill in the art to which this invention belongs and read in view of this disclosure.

In this document and in its claims, the verb "to comprise" and its conjugations is used in its non-limiting sense to mean that items following the word are included, but items not specifically mentioned are not excluded. In addition, the verb "to consist" may be replaced by "to consist essentially of" meaning that a method as described herein may comprise additional step(s) than the ones specifically identified, said additional step(s) not altering the unique characteristic of the invention. In addition, the verb "to consist" may be replaced by "to consist essentially of" meaning that a flavour composition, a heme or hemeprotein, a myoglobin, a gene construct, a host cell (or methods) as described herein may comprise additional component(s) (or additional steps) than the ones specifically identified, said additional component(s) not altering the unique characteristic of the invention.

Reference to an element by the indefinite article "a" or "an" does not exclude the possibility that more than one of the elements is present, unless the context clearly requires that there be one and only one of the elements. The indefinite article "a" or "an" thus usually means "at least one". As used herein, with "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. The word "about" or "approximately" when used in association with a numerical value (e.g. about 10) preferably means that the value may be the given value (of 10) more or less 0.1% of the value. As used herein, the term "and/or" indicates that one or more of the stated cases may occur, alone or in combination with at least one of the stated cases, up to with all of the stated cases.

In the context of this application, all percentages in the context of a concentration or composition refer to weight percentages, unless defined otherwise. In the context of this application, expressions such as "a parameter having a value of at least X, Y or Z" should be interpreted as said parameter having a value of at least X, of at least Y, or of at least Z.

Various embodiments are described herein. Each embodiment as identified herein may be combined together unless otherwise indicated. All patent applications, patents, and printed publications cited herein are incorporated herein by reference in the entireties, except for any definitions, subject matter disclaimers or disavowals, and except to the extent that the incorporated material is inconsistent with the express disclosure herein, in which case the language in this disclosure controls. One skilled in the art will recognize many methods and materials similar or equivalent to those described herein, which could be used in the practice of the present invention. Indeed, the present invention is in no way limited to the methods and materials described. The present invention is further described by the following examples which should not be construed as limiting the scope of the invention.

### Sequence identity

In the context of the invention, a nucleic acid molecule such as a nucleic acid molecule encoding an myoglobin is represented by a nucleic acid or nucleotide sequence which encodes a protein fragment or a polypeptide or a peptide or a derived peptide. It is to be understood that each nucleic acid molecule or protein fragment or polypeptide or peptide or derived peptide or construct as identified herein by a given sequence identity number (SEQ ID NO) is not limited to this specific sequence as disclosed. Each coding sequence as identified herein encodes a given protein fragment or polypeptide or peptide or derived peptide or construct or is itself a protein fragment or polypeptide or construct or peptide or derived peptide.

Throughout this application, each time one refers to a specific nucleotide sequence SEQ ID NO (take SEQ ID NO: X as example) encoding a given protein fragment or polypeptide or peptide or derived peptide, one may replace it by:
i. a nucleotide sequence comprising a nucleotide sequence that has at least 60% sequence identity with SEQ ID NO: X; or
ii. a nucleotide sequence the sequence of which differs from the sequence of a nucleic acid molecule of (i) due to the degeneracy of the genetic code; or
iii. a nucleotide sequence that encodes an amino acid sequence that has at least 60% amino acid identity or similarity with an amino acid sequence encoded by a nucleotide sequence SEQ ID NO: X.

Another preferred level of sequence identity or similarity is 70%. Another preferred level of sequence identity or similarity is 75%. Another preferred level of sequence identity or similarity is 80%. Another preferred level of sequence identity or similarity is 85%. Another preferred level of sequence identity or similarity is 90%. Another preferred level of sequence identity or similarity is 95%. Another preferred level of sequence identity or similarity is 99%.

Throughout this application, each time one refers to a specific amino acid sequence SEQ ID NO (take SEQ ID NO: Y as example), one may replace it by: a polypeptide represented by an amino acid sequence comprising a sequence that has at least 60% sequence identity or similarity with amino acid sequence SEQ ID NO: Y. Another preferred level of sequence identity or similarity is 70%. Another preferred level of sequence identity or similarity is 75%. Another preferred level of sequence identity or similarity is 80%. Another preferred level of sequence identity or similarity is 85%. Another preferred level of sequence identity or similarity is 90%. Another preferred level of sequence identity or similarity is 95%. Another preferred level of sequence identity or similarity is 99%.

Each nucleotide sequence or amino acid sequence described herein by virtue of its identity or similarity percentage with a given nucleotide sequence or amino acid sequence respectively has in a further preferred embodiment an identity or a similarity of at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% with the given nucleotide or amino acid sequence, respectively.

The terms "homology", "sequence identity" and the like are used interchangeably herein. Sequence identity is described herein as a relationship between two or more amino acid (polypeptide or protein) sequences or two or more nucleic acid (polynucleotide) sequences, as determined by comparing the sequences. In a preferred embodiment, sequence identity is calculated based on the full length of two given SEQ ID NO's or on a part thereof. Part thereof preferably means at least 50%, 60%, 70%, 80%, 90%, or 100% of both SEQ ID NO's. In the art, "identity" also refers to the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in Bioinformatics and the Cell: Modern Computational Approaches in Genomics, Proteomics and transcriptomics, Xia X., Springer International Publishing, New York, 2018; and Bioinformatics: Sequence and Genome Analysis, Mount D., Cold Spring Harbor Laboratory Press, New York, 2004, each incorporated herein by reference.

"Sequence identity" and "sequence similarity" can be determined by alignment of two peptide or two nucleotide sequences using global or local alignment algorithms, depending on the length of the two sequences. Sequences of similar lengths are preferably aligned using a global alignment algorithms (e.g. Needleman-Wunsch) which aligns the sequences optimally over the entire length, while sequences of substantially different lengths are preferably aligned using a local alignment algorithm (e.g. Smith-Waterman). Sequences may then be referred to as "substantially identical" or "essentially similar" when they (when optimally aligned by for example the program EMBOSS needle or EMBOSS water using default parameters) share at least a certain minimal percentage of sequence identity (as described below).

A global alignment is suitably used to determine sequence identity when the two sequences have similar lengths. When sequences have a substantially different overall length, local alignments, such as those using the Smith-Waterman algorithm, are preferred. EMBOSS needle uses the Needleman-Wunsch global alignment algorithm to align two sequences over their entire length (full length), maximizing the number of matches and minimizing the number of gaps. EMBOSS water uses the Smith-Waterman local alignment algorithm. Generally, the EMBOSS needle and EMBOSS water default parameters are used, with a gap open penalty = 10 (nucleotide sequences) / 10 (proteins) and gap extension penalty = 0.5 (nucleotide sequences) / 0.5 (proteins). For nucleotide sequences the default scoring matrix used is DNAfull and for proteins the default scoring matrix is Blosum62 (Henikoff & Henikoff, 1992, PNAS 89, 915-919, incorporated herein by reference).

Alternatively, percentage similarity or identity may be determined by searching against public databases, using algorithms such as FASTA, BLAST, etc. Thus, the nucleic acid and protein sequences of some embodiments of the present invention can further be used as a "query sequence" to perform a search against public databases to, for example, identify other family members or related sequences.

Such searches can be performed using the BLASTn and BLASTx programs (version 2.0) of Altschul, et al. (1990) J. Mol. Biol. 215:403-10, incorporated herein by reference. BLAST nucleotide searches can be performed with the NBLAST program, score = 100, wordlength = 12 to obtain nucleotide sequences homologous to oxidoreductase nucleic acid molecules of the invention. BLAST protein searches can be performed with the BLASTx program, score = 50, wordlength = 3 to obtain amino acid sequences homologous to protein molecules of the invention. To obtain gapped alignments for comparison purposes, Gapped BLAST can be utilized as described in Altschul et al., (1997) Nucleic Acids Res. 25(17): 3389-3402, incorporated herein by reference. When utilizing BLAST and Gapped BLAST programs, the default parameters of the respective programs (e.g., BLASTx and BLASTn) can be used. See the homepage of the National Center for Biotechnology Information accessible on the world wide web at www.ncbi.nlm.nih.gov/.

Optionally, in determining the degree of amino acid similarity, the skilled person may also take into account so-called conservative amino acid substitutions. As used herein, "conservative" amino acid substitutions refer to the interchangeability of residues having similar side chains. Examples of classes of amino acid residues for conservative substitutions are given in the Tables below.

| | |
|---|---|
| Acidic Residues | Asp (D) and Glu (E) |
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

Alternative conservative amino acid residue substitution classes:

| | | | |
|---|---|---|---|
| 1 | A | S | T |
| 2 | D | E | |
| 3 | N | Q | |
| 4 | R | K | |
| 5 | I | L | M |
| 6 | F | Y | W |

Alternative physical and functional classifications of amino acid residues:

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P and T |
| Flexible residues | Q, T, K, S, G, P, D, E, and R |

For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to Ser; Arg to Lys; Asn to Gln or His; Asp to Glu; Cys to Ser or Ala; Gln to Asn; Glu to Asp; Gly to Pro; His to Asn or Gln; Ile to Leu or Val; Leu to Ile or Val; Lys to Arg; Gln or Glu; Met to Leu or Ile; Phe to Met, Leu or Tyr; Ser to Thr; Thr to Ser; Trp to Tyr; Tyr to Trp or Phe; and, Val to lie or Leu.

### Proteins and amino acids

The terms "protein" or "polypeptide" or "amino acid sequence" are used interchangeably and refer to molecules consisting of a chain of amino acids, without reference to a specific mode of action, size, 3-dimensional structure or origin. In amino acid sequences as described herein, amino acids or "residues" are denoted by three-letter symbols. These three-letter symbols as well as the corresponding one-letter symbols are well known to a person of skill in the art and have the following meaning: A (Ala) is alanine, C (Cys) is cysteine, D (Asp) is aspartic acid, E (Glu) is glutamic acid, F (Phe) is phenylalanine, G (Gly) is glycine, H (His) is histidine, I (Ile) is isoleucine, K (Lys) is lysine, L (Leu) is leucine, M (Met) is methionine, N (Asn) is asparagine, P (Pro) is proline, Q (Gln) is glutamine, R (Arg) is arginine, S (Ser) is serine, T (Thr) is threonine, V (Val) is valine, W (Trp) is tryptophan, Y (Tyr) is tyrosine. A residue may be any proteinogenic amino acid, but also any non-proteinogenic amino acid such as D-amino acids and modified amino acids formed by post-translational modifications, and also any non-natural amino acid, as described herein.

### Gene or coding sequence

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and a 3'-nontranslated sequence (3'-end) e.g. comprising a polyadenylation- and/or transcription termination site. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide. As used herein, a "regulator" or "transcriptional regulator" is a protein that controls the rate of transcription of genetic information from DNA to messenger RNA, by binding to a specific DNA sequence.

### Promoter

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most physiological and developmental conditions. An "inducible" and/or "repressible" promoter is a promoter that is physiologically or developmentally regulated to be induced and/or repressed, e.g. by the application of a chemical inducer or repressing signal.

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame. Linking can be accomplished by ligation at convenient restriction sites or at adapters or linkers inserted in lieu thereof, or by gene synthesis.

### Gene constructs and expression vectors

Gene constructs as described herein could be prepared using any cloning and/or recombinant DNA techniques, as known to a person of skill in the art, in which a nucleotide sequence encoding said recombinant myoglobin is expressed in a suitable cell, e.g. cultured cells or cells of a multicellular organism, such as described in Ausubel et al., "Current Protocols in Molecular Biology", Greene Publishing and Wiley-Interscience, New York (1987) and in Sambrook and Russell (2001, *supra);* both of which are incorporated herein by reference in their entirety. Also see, Kunkel (1985) Proc. Natl. Acad. Sci. 82:488 (describing site directed mutagenesis) and Roberts et al. (1987) Nature 328:731-734 or Wells, J.A., et al. (1985) Gene 34: 315 (describing cassette mutagenesis).

The phrase "expression vector" or "vector" generally refers to a tool in molecular biology used to obtain gene expression in a cell, for example by introducing a nucleotide sequence that is capable of effecting expression of a gene or a coding sequence in a host compatible with such sequences. An expression vector carries a genome that is able to stabilize and remain episomal in a cell. Within the context of the invention, a cell may mean to encompass a cell used to make the construct or a cell wherein the construct will be administered. Alternatively, a vector is capable of integrating into a cell's genome, for example through homologous recombination or otherwise.

These expression vectors typically include at least suitable promoter sequences and optionally, transcription termination signals. An additional factor necessary or helpful in effecting expression can also be used as described herein. A nucleic acid or DNA or nucleotide sequence encoding a myoglobin is incorporated into a DNA construct capable of introduction into and expression in an *in vitro* cell culture. Specifically, a DNA construct is suitable for replication in a prokaryotic host, such as bacteria, *e.g., E. coli,* or can be introduced into a cultured mammalian, plant, insect, *(e.g.,* Sf9), yeast, fungi or other eukaryotic cell lines.

A DNA construct prepared for introduction into a particular host may include a replication system recognized by the host, an intended DNA segment encoding a desired polypeptide, and transcriptional and translational initiation and termination regulatory sequences operably linked to the polypeptide-encoding segment. The term "operably linked" has already been described herein. For example, a promoter or enhancer is operably linked to a coding sequence if it stimulates the transcription of the sequence. DNA for a signal sequence is operably linked to DNA encoding a polypeptide if it is expressed as a preprotein that participates in the secretion of a polypeptide. Generally, a DNA sequence that is operably linked are contiguous, and, in the case of a signal sequence, both contiguous and in reading frame. However, enhancers need not be contiguous with a coding sequence whose transcription they control. Linking is accomplished by ligation at convenient restriction sites or at adapters or linkers inserted in lieu thereof, or by gene synthesis.

The selection of an appropriate promoter sequence generally depends upon the host cell selected for the expression of a DNA segment. Examples of suitable promoter sequences include prokaryotic, and eukaryotic promoters well known in the art (see, *e.g.* Sambrook and Russell, 2001, *supra).* A transcriptional regulatory sequence typically includes a heterologous enhancer or promoter that is recognised by the host. The selection of an appropriate promoter depends upon the host, but promoters such as the trp, lac and phage promoters, tRNA promoters and glycolytic enzyme promoters are known and available *(see, e.g.* Sambrook and Russell, 2001, *supra).* An expression vector includes the replication system and transcriptional and translational regulatory sequences together with the insertion site for the polypeptide encoding segment. In most cases, the replication system is only functional in the cell that is used to make the vector (bacterial cell as *E. Coli*). Most plasmids and vectors do not replicate in the cells infected with the vector. Examples of workable combinations of cell lines and expression vectors are described in Sambrook and Russell (2001, *supra)* and in Metzger et al. (1988) Nature 334: 31-36. For example, suitable expression vectors can be expressed in, yeast, *e.g.* S. *cerevisiae, e.g.,* insect cells, *e.g.,* Sf9 cells, mammalian cells, *e.g.,* CHO cells and bacterial cells, *e.g., E. coli.* A cell may thus be a prokaryotic or eukaryotic host cell. A cell may be a cell that is suitable for culture in liquid or on solid media. Alternatively, a host cell is a cell that is part of a multicellular organism such as a transgenic plant or animal.

For example if a bacterium (preferably E. coli) is used as host cell, the following regulatory regions may be used. A promoter suitable to be used in a bacterium is lac, trp, tac, T7, phoA, ara, xapA, cad, recA, spc, bla, P1 and P2 from rrnB ribosomal RNA operon, PL promoter from phage λ. A terminator suitable to be used in a bacterium is lac, trp, tac, T7 (used in example), phoA, ara, xapA, cad, recA, spc, bla, P1 and P2 from rrnB ribosomal RNA operon, PL terminator from phage λ. A preferred promoter used is a T7 promoter and/or a preferred terminator is the T7 terminator. A preferred signal peptide for excretion is E. coli Sec-recognition peptide (SecA), E. coli Tet-recognition peptide, E. coli dsbA, E. coli phoA, E. coli pelB, E. coli MBP (maltose binding protein). A marker suitable for E coli is ampicillin. Alternatively, the proBA operon from E. coli strain K12, including its original transcription regulatory elements may be used to facilitate selection without antibiotics.

In some embodiments, the microorganisms do not comprise a selection marker or an antibiotic resistance marker. In an embodiment, the microorganism is a yeast. In an embodiment, the yeast is *Pichia pastoris.*

In another example, if a yeast is used as host cell, the following regulatory regions may be used. A promoter suitable to be used in yeast may be a constitutive promoter. Examples of suitable constitutive promoters include: a glycolytic promoter selected from FBA1, TPI1, PGK1, PYK1, TDH3, ENO2, HXK2, PGI1, PFK1, PFK2, GPM1 gene or a non-glycolytic promoter of the TEF2 gene. A suitable promoter to be used in yeast may be inducible. If the yeast is a *Pichia,* the methanol inducible promoter AOX1 is preferred. Otherwise the GAL1 promoter (galactose-inducible) may be used when the yeast is S. *cerevisiae.* The genes mentioned from which a promoter could be derived for a yeast as host cell could also be used to derive a terminator for the same yeast. A preferred signal peptide for excretion for *Pichia* (and *Saccharomyces)* includes: the S. *cerevisiae* alpha mating factor pre- pro- secretion signal peptide, the S. *cerevisiae* Ost1 signal peptide, the S. *cerevisiae* Aga2 signal peptide and fusions thereof.

In another example, if a filamentous fungus is used as host cell, the following regulatory regions may be used. The following promoters may be used: the *Aspergillus niger* glucoamylase promoter (glaA), the *Aspergillus nidulans* alcohol dehydrogenase promoter (alcA) or the *Aspergillus oryzae* taka-amylase A promoter *(amyB),* the *Aspergillus niger* alcohol dehydrogenase promoter *(acfM),* the *Trichoderma reesei* pyruvate kinase promoter *(pki)* or the *Aspergillus nidulas* glyceraldehyde-3-phosphate dehydrogenase promoter *(gpdA).* The genes mentioned from which a promoter could be derived for a filamentous fungus as host cell could also be used to derive a terminator for the same filamentous fungus. A preferred signal peptide for excretion for a filamentous fungus, preferably an *Aspergillus* includes: the *Aspergillus niger* glucoamylase signal peptide (glaA), the *Aspergillus niger* a-galactosidase signal peptide *(AgIB)* and the *Trichoderma reesei* cellobiohydrolase I (*Cbhl*). A preferred promoter and terminator for *Aspergillus* (more preferably for *Aspergillus niger)* are the glucoamylase promoter and the glucoamylase terminator of *Aspergillus niger.*

Gene constructs described herein can be placed in expression vectors. Thus, in another aspect there is provided an expression vector comprising a gene construct as described in any of the preceding embodiments.

Expression may be assessed by any method known to a person of skill in the art. For example, expression may be assessed by measuring the levels of transgene expression in the transduced tissue on the level of the mRNA or the protein by standard assays known to a person of skill in the art, such as qPCR, RNA sequencing, Northern blot analysis, Western blot analysis, mass spectrometry analysis of protein-derived peptides or ELISA. Expression may be assessed at any time after administration of the gene construct, expression vector or composition as described herein. In some embodiments herein, expression may be assessed after 1 week, 2 weeks, 3 weeks, 4, weeks, 5 weeks, 6 weeks, 7 weeks, 8 weeks, 9, weeks, 10 weeks, 11 weeks, 12 weeks, 14 weeks, 16 weeks, 18 weeks, 20 weeks, 22 weeks, 24 weeks, 28 weeks, 32 weeks, 36 weeks, 40 weeks, or more.

Suitable cell culturing methods for use in the process of producing recombinant protein are known to the skilled person, for example, in van't Riet, K. and Tramper, J., 1st edition, Basic Bioreactor Design, CRC Press, NY, 1991. Such methods include, but are not limited to, submerged fermentation in liquid media, surface fermentation on liquid media and solid-state fermentations. Cell culturing may, for example, be performed by cultivation in micro-titer plates, shake-flasks, small-scale benchtop bioreactors, medium-scale bioreactors and/or large-scale bioreactors in a laboratory and/or an industrial setting. Suitable cell culturing modes include, but are not limited to, continuous, batch and/or fed-batch fermentation as well as their combinations. Cell culturing may be performed using continuous fermentation, batch fermentation, preferably fed batch fermentation.

In the context of the invention, "culture medium", hereinafter alternately referred to as "growth medium", can be interpreted to encompass cases wherein the cultured cells are absent as well as cases wherein the cultured cells are present in the culture medium. "Culture broth" refers to the culture medium wherein the cultured cells are present. "Culture supernatant" refers to the culture medium wherein the cultured cells are absent. "Cell-free extract" refers to a cell lysate not comprising the cellular debris. Cell culturing as part of the process of the invention can be performed under conditions conducive to the production of the introduced myoglobins, which are known to the skilled person. Such conditions depend not only on the chemical composition of the culture medium but also on other process parameters including culture duration, temperature, O₂ levels in the culture broth and/or headspace, CO₂ levels in the culture broth and/or headspace, pH, ionic strength, agitation speed, hydrostatic pressure and the like. Cell culturing can take place using a culture medium comprising suitable nutrients, such as carbon and nitrogen sources and additional compounds such as inorganic salts and vitamins, using procedures known in the art (see, e. g. Bennett, W. and Lasure, L., 1st edition, More Gene Manipulations in Fungi, Academic Press, CA, 1991). Suitable growth media are available from commercial suppliers or may be prepared using published compositions that are suitable for the respective hosts (e.g. in catalogues of the Centraalbureau Voor Schimmelcultures collection (CBS) or of the American Type Culture Collection (ATCC)).

The exact composition of the growth medium and the values of culture process parameters are not critical features of the invention. Any growth medium composition may be contemplated, as long as it allows for growth of the host cell and production of the introduced myoglobins. The growth medium will typically comprise a carbon source to be used for the growth of the cultured cell. The skilled person understands that suitable carbon sources may be added externally to the growth medium or may already be present in said medium. Carbon sources may be present or added individually or in mixtures of multiple carbon sources. Examples of suitable carbon sources known in the art include simple sugars such as glucose, glycerol, maltose, sucrose, xylose, arabinose, complex sugars such as maltodextrins, hydrolysed starch, starch, molasses, and second-generation feedstocks. Second-generation feedstocks can be particularly attractive because of their lower carbon footprint. Second-generation feedstocks will typically comprise lignocellulosic material. Such material includes any lignocellulose and/or hemicellulose-based materials. Such material may be sourced from agricultural, industrial or municipal, preferably agricultural, waste streams. Examples of suitable materials include (agricultural) biomass, commercial organic matter, municipal solid waste, virgin biomass such as waste paper and garden waste, or non-virgin biomass. General forms of biomass include trees, shrubs and pastures, wheat, wheat straw, sugarcane bagasse, switchgrass, Japanese pampas grass, corn, corn stover, corn cob, canola stalk, soybean stalk, sweet corn, corn kernels, products and by-products from cereal milling (including wet milling and dry milling), such as corn, wheat, and barley, often referred to as "bran or fiber", and municipal solids. Biomass can also be grassy materials, agricultural residues, forestry residues, municipal solid waste, wastepaper, and pulp and paper mill residues. Agricultural biomass includes branches, shrubs, tows, corn and corn straw, energy crops, forests, fruits, flowers, cereals, pastures, herbaceous crops, leaves, bark, needles, logs, roots, young trees, short term rotating woody crops, shrubs, switch herbs, trees, vegetables, fruits, vines, sugar beet pulp, wheat middlings, oat hulls, and hard and soft timber (not including toxic wood), and organic waste materials resulting from agricultural processes including agriculture and forestry activities, particularly forestry wood waste. Agricultural biomass may be any of the foregoing alone, or any combination or mixture thereof. Carbon sources such as organic acids, aldehydes, ketones, esters and alcohols may also be contemplated. The use of growth media comprising combinations of multiple different carbon sources may also be contemplated in the process of the invention. Such media could, as a non-limiting example, combine more oxidized carbon sources such as organic acids with more reduced carbon sources such as alcohols. Examples of suitable nitrogen sources known in the art include soybean meal, corn steep liquor, yeast extract, whey protein, egg protein, casein hydrolysate, urea, ammonia, ammonium salts and nitrate salts. Examples of additional suitable compounds known in the art include phosphate, sulphate, metals such as magnesium, trace elements and vitamins. The exact growth medium requirements will vary based on the host cell, e.g. between yeasts, bacteria, and filamentous fungi, said requirements will be known to the skilled person. Accordingly, the growth medium may be a complete (rich) medium or a minimal medium, i.e. a medium comprising only the absolutely necessary components for growth depending on the cultured host cell.

Similar to the composition of the growth medium, process parameters can be assigned any value, as long as they allow for growth of the host cell and production of the introduced myoglobins. Typically, said values will differ based on the host cell that is being cultured and will be known to the skilled person. Preferably, the process according to the invention is an oxygen-limited or aerobic process, meaning that cell culturing is performed under oxygen-limited or aerobic conditions, more preferably the process is oxygen-limited. Oxygen-limited conditions, also known as a micro-aerobic conditions, are culture conditions in which the oxygen consumption is limited by the availability of oxygen. The degree of oxygen limitation is determined by the amount and composition of the ingoing gas flow as well as the actual mixing/mass transfer properties of the fermentation equipment used. Preferably, under oxygen-limited conditions in a liquid culture, the rate of oxygen consumption is at least about 5.5 mmol/L/h, more preferably at least about 6 mmol/L/h and even more preferably at least about 7 mmol/L/h. Aerobic conditions are culture conditions in which the oxygen consumption is not limited by the availability of oxygen.

Cell culturing may be performed at a temperature value that is optimal for the cell, typically at a temperature range of 16-42 °C. In some embodiments, the temperature ranges between 20-40 °C, more preferably between 25-38 °C, most preferably between 28-36 °C. In some most preferred embodiments, a temperature value of about 30 or 36 °C is used.

Cell culturing may be performed at a pH value that is optimal for the cell. In some embodiments, the culture pH value is about pH 2.5, about pH 3.0, about pH 3.5, about pH 4.0, about pH 4.5, about pH 5, about pH 5.5, about pH 6, about pH 6.5, about pH 7, about pH 7.5, about pH 8.0, about pH 8.5, about pH 9. In preferred embodiments, the pH ranges from about pH 3.0 to about pH 9, more preferably from about pH 3.5 to pH 7. In some most preferred embodiments, a pH value of about 6 is used.

Cell culturing may be performed at an ionic strength value of the culture medium that is optimal for the cell, typically at a range between 50 mM - 2 M. In some embodiments, the ionic strength of the culture medium ranges between 75 mM - 1 M, more preferably between 100 mM - 750 mM. In some most preferred embodiments, an ionic strength value of about 100 mM is used.

### Overview of the sequence listing

| Protein | SEQ ID NO protein | SEQ ID NO coding nucleic acid |
|---|---|---|
| Steppe mammoth myoglobin | 1 | 15 |
| Woolly mammoth myoglobin | 2 | 16 |
| Pig myoglobin | 3 | 17 |
| Sheep myoglobin | 4 | 18 |
| Cow myoglobin | 5 | 19 |
| Chicken myoglobin | 6 | 20 |
| Rabbit myoglobin | 7 | 21 |
| Bovine myoglobin | 8 | 22 |
| Mouse myoglobin | 9 | 23 |
| Rat myoglobin | 10 | 24 |
| Tuna myoglobin | 11 | 25 |
| Salmon myoglobin | 12 | 26 |
| Blue bonito myoglobin | 13 | 27 |
| Sword fish myoglobin | 14 | 28 |

### Detailed sequences

- SEQ ID NO:1 Steppe mammoth myoglobin 
MGLSDGEWELVLKTWGKVEADIPGHGLEVFVRLFTGHPETLEKFDKFKHLKTEGEMKASEDLKK
QGVTVLTALGGILKKKGHHQAEIQPLAHSHATKHKIPIKYLEFISDAIIHVLQSKHPAEFGADAQGA
MKKALELFRNDIAAKYKELGFQG
- SEQ ID NO:2 Woolly mammoth myoglobin 
MGLSDGEWELVLKTWGKVEADIPGHGLEVFVRLFTGHPETLEKFDKFKHLKTEGEMKASEDLKK
QGVTVLTALGGILKKKGHHQAEIQPLAQSHATKHKIPIKYLEFISDAIIHVLQSKHPAEFGADAQGA
MKKALELFRNDIAAKYKELGFQG
- SEQ ID NO:3 Pig myoglobin 
MGLSDGEWQLVLNVWGKVEADVAGHGQEVLIRLFKGHPETLEKFDKFKHLKSEDEMKASEDLK
KHGNTVLTALGGILKKKGHHEAELTPLAQSHATKHKIPVKYLEFISEAIIQVLQSKHPGDFGADAQ
GAMSKALELFRNDMAAKYKELGFQG
- SEQ ID NO:4 Sheep myoglobin 
MGLSDGEWQLVLNAWGKVEADVAGHGQEVLIRLFTGHPETLEKFDKFKHLKTEAEMKASEDLK
KHGNTVLTALGGILKKKGHHEAEVKHLAESHANKHKIPVKYLEFISDAIIHVLHAKHPSDFGADAQ
GAMSKALELFRNDMAAQYKVLGFQG
- SEQ ID NO:5 Cow myoglobin 
MGLSDGEWQLVLNAWGKVEADVAGHGQEVLIRLFTGHPETLEKFDKFKHLKTEAEMKASEDLK
KHGNTVLTALGGILKKKGHHEAEVKHLAESHANKHKIPVKYLEFISDAIIHVLHAKHPSDFGADAQA
AMSKALELFRNDMAAQYKVLGFHG
- SEQ ID NO:6 Chicken myoglobin 
MGLSDQEWQQVLTIWGKVEADIAGHGHEVLMRLFHDHPETLDRFDKFKGLKTPDQMKGSEDLK
KHGATVLTQLGKILKQKGNHESELKPLAQTHATKHKIPVKYLEFISEVIIKVIAEKHAADFGADSQA
AMKKALELFRNDMASKYKEFGFQG
- SEQ ID NO:7 Rabbit myoglobin 
MGLSDAEWQLVLNVWGKVEADLAGHGQEVLIRLFHTHPETLEKFDKFKHLKSEDEMKASEDLKK
HGNTVLTALGAILKKKGHHEAEIKPLAQSHATKHKIPVKYLEFISEAIIHVLHSKHPGDFGADAQAA
MSKALELFRNDIAAQYKELGFQG
- SEQ ID NO:8 Bovine myoglobin 
MGLSDGEWQLVLNAWGKVEADVAGHGQEVLIRLFTGHPETLEKFDKFKHLKTEAEMKASEDLK
KHGNTVLTALGGILKKKGHHEAEVKHLAESHANKHKIPVKYLEFISDAIIHVLHAKHPSDFGADAQA
AMSKALELFRNDMAAQYKVLGFHG
- SEQ ID NO:9 Mouse myoglobin 
MGLSDGEWQLVLNVWGKVEADLAGHGQEVLIGLFKTHPETLDKFDKFKNLKSEEDMKGSEDLK
KHGCTVLTALGTILKKKGQHAAEIQPLAQSHATKHKIPVKYLEFISEIIIEVLKKRHSGDFGADAQGA
MSKALELFRNDIAAKYKELGFQG
- SEQ ID NO:10 Rat myoglobin 
MGLSDGEWQMVLNIWGKVEGDLAGHGQEVLISLFKAHPETLEKFDKFKNLKSEEEMKSSEDLKK
HGCTVLTALGTILKKKGQHAAEIQPLAQSHATKHKIPVKYLEFISEVIIQVLKKRYSGDFGADAQGA
MSKALELFRNDIAAKYKELGFQG
- SEQ ID NO:11 Tuna myoglobin 
MADFDAVLKCWGPVEADYTTIGGLVLTRLFKEHPETQKLFPKFAGIAQADIAGNAAVSAHGATVL
KKLGELLKAKGSHAAILKPLANSHATKHKIPINNFKLISEVLVKVMHEKAGLDAGGQTALRNVMGIII
ADLEANYKELGFSG
- SEQ ID NO:12 Salmon myoglobin 
MANYDMVLQCWEPVEADYNNHGGLVLSRLFAEHPETLTLFPKFAGIAAGDLSGNAAVAAHGATV
LRKLGELLNARGDHAATLKSLATTHANKHKIPLKNFTLITNIICKVMGEKAGLDEAGQEALRQVMG
VIIADINVTYMELGFAG
- SEQ ID NO:13 Blue bonito myoglobin 
ADFDAVLKFWGPVEADYTSHGGLVLTRLFKEHPETQKLFPKFTGIAQADMAGNAAISAH
GATVLKKLGELLKAKGNHAAILKPMANSHATKHKIPINNFKLISEIIVKVMQEKAGMDAG
GQQALRNVMAAVIADLEANYKELGFSG
- SEQ ID NO:14 Sword fish myoglobin

MAEFEVVLKHWGPVEADYASHGNLVLTRLFTEHPDTQKLFPKFAGIAQGDMAGNAAISAHGATV
LKKLGELLKAKGNHATTLKALATTHATKHKIPIKNFELIAEVIGKVMEEKAGLDSVGREALRDVMAR
VIADIDATYKELGFTG

## Claims

1. A flavour composition comprising a hemeprotein for modifying the flavour of a foodstuff or a beverage.

2. The flavour composition according to claim 1, wherein the hemeprotein is a hemeprotein obtained from microbial fermentation.

3. The flavour composition according to claim 2, wherein said flavour composition comprises at least part of the fermentation broth or part of the microbial host.

4. The flavour composition according to claim 2 or 3, wherein the hemeprotein is extracellularly secreted from the microbial host, and/or wherein the hemeprotein is recombinant hemeprotein.

5. The flavour composition according to claims 1-4, wherein the hemeprotein is a myoglobin.

6. The flavour composition according to claim 5, wherein the myoglobin is a myoglobin from steppe mammoth, woolly mammoth, pig, sheep, cow, chicken, rabbit, bovine, mouse, rat, tuna, salmon, blue bonito, or sword fish.

7. The flavour composition according to any one of claims 5-6, wherein the myoglobin is represented by a sequence having at least 95% identity with any one of SEQ ID NO: 1 to 14.

8. The flavour composition according to any of the preceding claims, wherein the composition further comprises a carrier acceptable for food, and/or one or more additives.

9. Use of the flavour composition as defined in any one of claims 1-8 for improving the flavour of a foodstuff or a beverage.

10. The flavour composition according to claims 1-8 or the use according to claim 8, wherein the foodstuff is not a meat or a meat analogue.

11. The flavour composition according to claims 1-8, and 10 or the use according to claim 9 or 10, wherein the foodstuff is a bouillon.

12. Method for improving the flavour of a foodstuff or a beverage, comprising adding to said foodstuff or beverage one or more flavour compositions as defined in any one of claims 1-8 or 10.

13. Method for preparing a flavour composition according to any one of claims 1-8 or 10, wherein the hemeprotein is a hemeprotein obtained from microbial fermentation by a microorganism, and/or wherein the hemeprotein is recombinant hemeprotein.

14. Method for preparing a flavour composition according to claim 13, wherein the microorganism is a bacterium, a yeast, or a filamentous fungus, preferably *Pichia pastoris.*
